# EUROPEAN PATENT SPECIFICATION

(11) **EP 4 641 573 B1**
(45) Date of publication and mention of the grant of the patent: **18.11.2026**
(21) Application number: 24210083.2
(22) Date of filing: 31.10.2024
(51) Int. Cl.: G16B 30/00

(54) **METHOD AND APPARATUS FOR DETECTING CHROMOSOMAL ANEUPLOIDY, DEVICE AND STORAGE MEDIUM**
VERFAHREN UND VORRICHTUNG ZUM NACHWEIS VON CHROMOSOMALER ANEUPLOIDIE, VORRICHTUNG UND SPEICHERMEDIUM
PROCÉDÉ ET APPAREIL DE DÉTECTION D'UNE ANEUPLOÏDIE CHROMOSOMIQUE, DISPOSITIF ET SUPPORT DE STOCKAGE

(30) Priority: 28.04.2024 CN 202410516920
(43) Date of publication of application: 29.10.2025
(73) Proprietor: GeneMind Biosciences Company Limited, Shenzhen City, Guangdong 518023 (CN)
(72) Inventor: LIU, Xianke, Shenzhen City, 518023 (CN)
(74) Representative: Novagraaf Group

(56) References cited:
- AU-A1- 2019 200 162
- US-A1- 2021 238 669
- FRANCESCO FIORENTINO ET AL: "The importance of determining the limit of detection of non-invasive prenatal testing methods : The importance of determining the limit of detection of NIPT methods", PRENETAL DIAGNOSIS, vol. 36, no. 4, 21 February 2016 (2016-02-21), GB, pages 304 - 311, XP055473015, ISSN: 0197-3851, DOI: 10.1002/pd.4780

## Description

### FIELD

The present invention relates to the field of biotechnology and, in particular, to a method and apparatus for detecting chromosomal aneuploidy, a device and a storage medium.

### BACKGROUND

Genome sequencing is applied to chromosomal aneuploidy screening services due to technical advantages such as good detection performance, a short period and non-invasiveness.

Presently, methods for detecting chromosomal aneuploidy mainly include a z-score algorithm, normalized chromosome values (NCVs) and a genome-wide normalized score (GWNS). The above detection methods have different indicators for determining chromosomal aneuploidy, and in most of these methods, whether a sequencing indicator of a chromosome in a sample under test deviates from the indicator distribution of the chromosome in a normal sample is determined so that whether the chromosome in the sample under test is an aneuploidy is determined.

In the above detection methods, parameters related to environment of the sample under test, for example, sample collection, a sequencing environment and a computing environment, are required to be consistent with those of the normal sample. However, due to the effects of factors such as limitations in hardware conditions in different scenarios and operation habits of operators, the indicator of the sample deviates from the indicator distribution of a normal sample set, resulting in a false positive result or even a false negative result. To improve the matching between the indicator of the sample and the indicator of the normal sample set, a large amount of time and resources often need to be consumed. Therefore, the above detection methods have relatively high detection and maintenance costs.

### PRIOR ART

AU 2019 200 162 A1 (VERINATA HEALTH INC [US]) 31 January 2019 (2019-01-31) discloses a method for classifying copy number variations, including fetal aneuploidies, by sequencing cell-free DNA from a maternal test sample, aligning sequence reads to bins of a reference genome, and comparing normalized tag counts between chromosomes or chromosome segments. US 2021/238669 A1 (VAN DEN BOOM DIRK JOHANNES [US] ET AL) 5 August 2021 (2021-08-05) discloses methods for detecting genetic variations, including chromosomal aneuploidy, by sequencing circulating cell-free nucleic acids from a test sample, mapping partial sequence reads to bins of a reference genome, and comparing normalized read counts for a test chromosome to reference values using statistical analysis. The approach emphasizes normalization (e.g., for GC content) and uses parametric statistical tests (such as z-scores) to classify the presence or absence of aneuploidy.

### SUMMARY

Embodiments of the present invention provide a method and apparatus for detecting chromosomal aneuploidy, a device and a storage medium to solve the problem of dependence of a method for detecting chromosomal aneuploidy on indicator distribution in a normal sample, thereby reducing detection and maintenance costs of chromosomal aneuploidy on the basis of relatively high accuracy.

An embodiment of the present invention provides a method for detecting chromosomal aneuploidy. The method includes the steps below.

A chromosome bin sequence of a chromosome under test is determined according to reference genome nucleic acid data of a human reference genome, where the chromosome bin sequence includes at least one bin number ratio, and each of the at least one bin number ratio is the ratio of the number of nucleic acid bins of the chromosome under test in the human reference genome to the number of nucleic acid bins of a respective one of at least one preset chromosome in the human reference genome.

A sequencing depth sequence of the chromosome under test is determined according to whole genome sequencing data of a nucleic acid sample under test, where the sequencing depth sequence includes at least one sequencing depth parameter, and each of the at least one sequencing depth parameter represents a functional relationship between a sequencing depth of the chromosome under test in the nucleic acid sample under test and a sequencing depth of a respective one of the at least one preset chromosome in the nucleic acid sample under test.

According to the chromosome bin sequence and the sequencing depth sequence, a non-parametric test is performed so that an aneuploidy detection result of the chromosome under test in the nucleic acid sample under test is determined.

Another embodiment of the present invention provides an apparatus for detecting chromosomal aneuploidy. The apparatus includes a chromosome bin sequence determination module, a sequencing depth sequence determination module and an aneuploidy detection result determination module.

The chromosome bin sequence determination module is configured to determine a chromosome bin sequence of a chromosome under test according to reference genome nucleic acid data of a human reference genome, where the chromosome bin sequence includes at least one bin number ratio, and each of the at least one bin number ratio is the ratio of the number of nucleic acid bins of the chromosome under test in the human reference genome to the number of nucleic acid bins of a respective one of at least one preset chromosome in the human reference genome.

The sequencing depth sequence determination module is configured to determine a sequencing depth sequence of the chromosome under test according to whole genome sequencing data of a nucleic acid sample under test, where the sequencing depth sequence includes at least one sequencing depth parameter, and each of the at least one sequencing depth parameter represents a functional relationship between a sequencing depth of the chromosome under test in the nucleic acid sample under test and a sequencing depth of a respective one of the at least one preset chromosome in the nucleic acid sample under test.

The aneuploidy detection result determination module is configured to, according to the chromosome bin sequence and the sequencing depth sequence, perform a non-parametric test to obtain an aneuploidy detection result of the chromosome under test in the nucleic acid sample under test.

Another embodiment of the present invention provides an electronic device. The electronic device includes the following components.

At least one processor is provided.

A memory communicatively connected to the at least one processor is also provided.

The memory stores a computer program executable by the at least one processor, and the computer program is executed by the at least one processor to cause the at least one processor to perform the method for detecting chromosomal aneuploidy according to any embodiment of the present invention.

Another embodiment of the present invention provides a computer-readable storage medium. The computer-readable storage medium stores a computer instruction, where the computer instruction, when executed by a processor, causes the processor to perform the method for detecting chromosomal aneuploidy according to any embodiment of the present invention.

According to the technical solutions of the embodiments of the present invention, the chromosome bin sequence built according to the human reference genome is used as a reference sequence of the chromosome under test, and the non-parametric test is performed according to the chromosome bin sequence and the sequencing depth sequence corresponding to the nucleic acid sample under test by using a correlation between a chromosome bin sequence and a sequencing depth sequence of a chromosome in nucleic acid data of euploidies so that the aneuploidy detection result of the chromosome under test in the nucleic acid sample under test is determined. The method has relatively high detection accuracy and solves the problem of the dependence of the method for detecting chromosomal aneuploidy on the indicator distribution in the normal sample so that a process of detecting chromosomal aneuploidy is no longer limited by a requirement for consistency between environmental parameters, and the detection and maintenance costs of chromosomal aneuploidy are reduced.

### BRIEF DESCRIPTION OF DRAWINGS

In order that the technical solutions in embodiments of the present invention are illustrated more clearly, the drawings used in the description of the embodiments are described briefly below. Apparently, the drawings described below illustrate only some embodiments of the present invention. Those of ordinary skill in the art may obtain other drawings based on these drawings on the premise that no creative work is done.
FIG. 1 is a flowchart of a method for detecting chromosomal aneuploidy according to an embodiment of the present invention;
FIG. 2 is another flowchart of a method for detecting chromosomal aneuploidy according to an embodiment of the present invention;
FIG. 3 is a flowchart of an example of a method for detecting chromosomal aneuploidy according to an embodiment of the present invention;
FIG. 4 is a structure diagram of an apparatus for detecting chromosomal aneuploidy according to an embodiment of the present invention; and
FIG. 5 is a structure diagram of an electronic device according to an embodiment of the present invention.

### DETAILED DESCRIPTION

For a better understanding of the solutions of the present invention by those skilled in the art, the technical solutions in embodiments of the present invention are described clearly and completely below in conjunction with the drawings in the embodiments of the present invention. Apparently, the embodiments described below are part, not all, of the embodiments of the present invention. Based on the embodiments of the present invention, all other embodiments obtained by those of ordinary skill in the art without creative work are within the scope of the present invention.

It is to be noted that terms such as "first", "second", "under test" and "preset" in the description, claims and above drawings of the present invention are used for distinguishing between similar objects and are not necessarily used for describing a particular order or sequence. It is to be understood that the data used in this manner are interchangeable where appropriate so that the embodiments of the present invention described herein may be implemented in a sequence not illustrated or described herein. Additionally, the term "including", "having" or any variation thereof is intended to encompass a non-exclusive inclusion. For example, a process, method, system, product or device that includes a series of steps or units not only includes the expressly listed steps or units but may also include other steps or units that are not expressly listed or are inherent to such process, method, product or device.

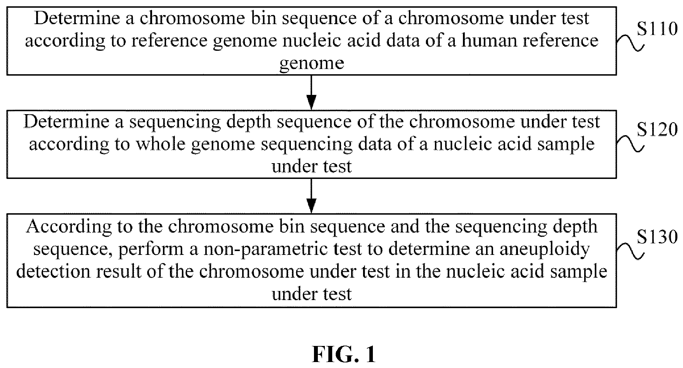

FIG. 1 is a flowchart of a method for detecting chromosomal aneuploidy according to an embodiment of the present invention. This embodiment is applicable to the detection of whether an aneuploidy exists among chromosomes in a nucleic acid sample. The method may be performed by an apparatus for detecting chromosomal aneuploidy. The apparatus for detecting chromosomal aneuploidy may be implemented by hardware and/or software and may be configured in a terminal device. As shown in FIG. 1, the method includes S110, S120 and S130.

In S110, a chromosome bin sequence of a chromosome under test is determined according to reference genome nucleic acid data of a human reference genome.

For example, a source of the human reference genome may include National Center for Biotechnology Information (NCBI) database version Genome Reference Consortium Human Build 36 (GRCh36), GRCh37 or GRCh38, University of California, Santa Cruz (UCSC) database version human genome 18 (hg18), hg19 or hg38. The source of the human reference genome is not limited herein and may be customized according to actual requirements.

In embodiments of the present application, nucleic acid data are used for representing nucleic acid sequences and may be standard sequences of the human reference genome (for example, the reference genome nucleic acid data) or sequences of a nucleic acid sample obtained through sequencing (for example, whole genome sequencing data). For example, the reference genome nucleic acid data in the embodiments of the present application refer to the standard sequences of the human reference genome, that is, sequences corresponding to real sequences of the human reference genome. For example, the reference genome nucleic acid data include at least a chromosome nucleic acid datum of the chromosome under test and a chromosome nucleic acid datum of each of at least one preset chromosome. The chromosome under test is used for representing a human chromosome detected for the aneuploidy, and each preset chromosome is used for representing another human chromosome excluding the chromosome under test. In the embodiments of the present application, each chromosome under test corresponds to a group of preset chromosomes, and characteristic data of the chromosome under test are acquired based on the group of preset chromosomes, such as the number of bins and a sequencing depth. For each chromosome under test, the selection of the preset chromosomes is not strictly limited and may be set according to a target requirement to be met in the detection and based on the method according to the embodiments of the present application. For example, the chromosome under test may be chromosome 21, and the preset chromosomes include chromosome 1, chromosome 2 and chromosome 3.

In an exemplary embodiment, the chromosome bin sequence represents a proportional function model of nucleic acid bins of the chromosome under test and the group of preset chromosomes in the human reference genome. In this embodiment, the chromosome bin sequence includes at least one bin number ratio, and each bin number ratio is the ratio of the number of nucleic acid bins of the chromosome under test in the human reference genome to the number of nucleic acid bins of one respective preset chromosome in the human reference genome.

In an exemplary embodiment, the number of nucleic acid bins may be used for representing the number of nucleic acid bins included in the chromosome nucleic acid datum of the chromosome under test or the preset chromosome in the human reference genome. Bin division is performed on the chromosome nucleic acid datum according to a bin division rule so that the nucleic acid bins are obtained, and a bin position of each nucleic acid bin in the chromosome nucleic acid datum is unique.

In an optional embodiment, that the chromosome bin sequence of the chromosome under test is determined according to the reference genome nucleic acid data of the human reference genome includes: acquiring, from the reference genome nucleic acid data, a reference chromosome nucleic acid datum of the chromosome under test and a reference chromosome nucleic acid datum of each of the at least one preset chromosome; for each reference chromosome nucleic acid datum, performing the bin division on the reference chromosome nucleic acid datum according to the bin division rule, and determining, according to a bin division result, the number of nucleic acid bins of the chromosome under test and the number of nucleic acid bins of each preset chromosome; and determining the chromosome bin sequence of the chromosome under test according to the number of nucleic acid bins of the chromosome under test and the number of nucleic acid bins of each preset chromosome.

In an exemplary embodiment, the reference chromosome nucleic acid datum is a nucleic acid sequence datum corresponding to the chromosome under test or a nucleic acid sequence datum corresponding to the preset chromosome in the human reference genome. For example, assuming that the chromosome under test is chromosome 18, the reference chromosome nucleic acid datum is a nucleic acid sequence datum corresponding to chromosome 18 in the reference genome nucleic acid data of the human reference genome.

In an optional embodiment, the bin division rule includes a preset bin length and an interval between bins, where the preset bin length is used for representing a bin sequence length of a nucleic acid bin obtained through division. A specific parameter value of the preset bin length is not limited herein and may be customized according to the actual requirements. For example, the preset bin length is, but is not limited to, 20 kbp.

In an exemplary embodiment, the interval between bins is used for representing the length of a nucleic acid sequence between two adjacent nucleic acid bins. For example, the interval between bins may be -1 kb, 0 kb or 1 kb, where "-1 kb" indicates that two adjacent nucleic acid bins have an overlap of a nucleic acid sequence of 1 kb, "0 kb" indicates that no nucleic acid sequence exists as an overlap or interval between two adjacent nucleic acid bins, and "1 kb" indicates that a nucleic acid sequence of 1 kb exists as an interval between two adjacent nucleic acid bins. A specific parameter value of the interval between bins is not limited herein and may be customized according to the actual requirements.

In an optional embodiment, according to the bin division result determining the number of nucleic acid bins of the chromosome under test and the number of nucleic acid bins of each preset chromosome includes: performing a deletion operation on a nucleic acid bin not including any known bases in the bin division result; and counting remaining nucleic acid bins in the bin division result after the deletion operation to obtain the number of nucleic acid bins of the chromosome under test and the number of nucleic acid bins of each preset chromosome.

In an exemplary embodiment, nucleic acid bins in the bin division result are traversed. If the nucleic acid bin does not include any known bases, it indicates that the nucleic acid bin includes all unknown bases, and the nucleic acid bin is deleted from the bin division result.

Such setting has the following advantage: the nucleic acid bin including all the unknown bases is prevented from causing noise interference to the accuracy of the number of nucleic acid bins counted subsequently and the sequencing depth, further ensuring the accuracy of an aneuploidy detection result.

For example, the bin number ratio of the chromosome under test i and the preset chromosome *j* may be represented as rᵢⱼ = Lᵢ/Lⱼ, where *i* ≠ *j,* Lᵢ denotes the number of nucleic acid bins of the chromosome under test i, and Lⱼ denotes the number of nucleic acid bins of the preset chromosome j. For example, a chromosome bin sequence R₁ of chromosome 1 may be represented as R₁ = [r₁₂, r₁₃, r₁₄, ..., r₁ⱼ].

In S120, a sequencing depth sequence of the chromosome under test is determined according to whole genome sequencing data of a nucleic acid sample under test.

For example, a type of the nucleic acid sample under test is not strictly limited and may be any one including complete human DNA, where complete DNA refers to DNA that is not damaged in a sampling process and after sampling. For example, the nucleic acid sample under test may be a blood sample, a urine sample, a cell sample, a mucus sample or a tissue sample. A source of the nucleic acid sample under test has no effect on the method for detecting chromosomal aneuploidy and a detection result of chromosomal aneuploidy. Therefore, the source of the nucleic acid sample under test is not limited in the embodiments of the present application and may be customized according to the actual requirements.

In the embodiments of the present application, the whole genome sequencing data of the nucleic acid sample under test are nucleic acid sequence data obtained after whole genome sequencing is performed on the nucleic acid sample under test. For example, the whole genome sequencing data include a chromosome sequencing datum of the chromosome under test and a chromosome sequencing datum of each of the at least one preset chromosome. The chromosome sequencing datum represents all nucleic acid data included in a chromosome in the unit of chromosome.

In an optional embodiment, the whole genome sequencing data of the nucleic acid sample under test may be obtained by a method including extracting a free nucleic acid from the nucleic acid sample under test; performing polymerase chain reaction (PCR) amplification on the free nucleic acid and performing sample pretreatment to obtain a nucleic acid library; and performing the whole genome sequencing on the nucleic acid library to obtain the whole genome sequencing data of the nucleic acid sample under test.

For example, the PCR amplification is performed on the free nucleic acid by using a PCR nucleic acid amplifier, and the nucleic acid library is built according to the amplified free nucleic acid by using a chromosomal aneuploidy detection kit. A sequencing technology used for the whole genome sequencing includes, but is not limited to, a second-generation sequencing technology, a nanopore sequencing technology or a third-generation sequencing technology. The sequencing technology used for the whole genome sequencing is not limited herein and may be customized according to the actual requirements.

In an exemplary embodiment, the sequencing depth sequence represents a function model of sequencing depths of the chromosome under test and the group of preset chromosomes in the nucleic acid sample under test. In this embodiment, the sequencing depth sequence includes at least one sequencing depth parameter, and each sequencing depth parameter represents a functional relationship between a sequencing depth of the chromosome under test in the nucleic acid sample under test and a sequencing depth of one respective preset chromosome in the nucleic acid sample under test. The sequencing depth refers to the number of unique alignment sequences of the nucleic acid sample under test detected in an area of the human reference genome.

In an optional embodiment, that the sequencing depth sequence of the chromosome under test is determined according to the whole genome sequencing data of the nucleic acid sample under test includes: acquiring, from the whole genome sequencing data, the chromosome sequencing datum of the chromosome under test and the chromosome sequencing datum of each of the at least one preset chromosome; for each chromosome sequencing datum, performing sequence alignment on the chromosome sequencing datum and at least one nucleic acid bin of a respective chromosome, determining the number of nucleic acid sequences in an alignment datum of each nucleic acid bin, and using the number of nucleic acid sequences in alignment data of the at least one nucleic acid bin as a sequencing depth of the respective chromosome; and determining the sequencing depth sequence of the chromosome under test according to the sequencing depth of the chromosome under test and a sequencing depth of each preset chromosome.

In an exemplary embodiment, the chromosome sequencing datum is a nucleic acid sequence datum corresponding to the chromosome under test or a nucleic acid sequence datum corresponding to the preset chromosome in the nucleic acid sample under test.

For example, assuming that the chromosome under test is chromosome 18, the chromosome sequencing datum is a nucleic acid sequence datum corresponding to chromosome 18 in the whole genome sequencing data of the nucleic acid sample under test, and the sequence alignment is performed on the chromosome sequencing datum of chromosome 18 and a nucleic acid bin of chromosome 18, where the nucleic acid bin of chromosome 18 is a nucleic acid bin counted to obtain the number of nucleic acid bins in S110.

For example, an alignment tool used in the alignment operation includes, but is not limited to, a Torrent Mapping Alignment Program (TMAP) tool, a Burrows-Wheeler Alignment (BWA) tool, a Short Oligonucleotide Alignment Program (SOAP) tool or Sequence Alignment/Map tools (SAMtools). The alignment tool used in the alignment operation is not limited herein and may be customized according to the actual requirements.

In an exemplary embodiment, the number of nucleic acid sequences is the number of nucleic acid fragments in each chromosome sequencing datum and aligned to a specified nucleic acid bin and may represent the distribution of the nucleic acid fragments in the specified nucleic acid bin.

In an optional embodiment, determining the number of nucleic acid sequences in the alignment datum of each nucleic acid bin includes: acquiring an initial number of sequences in the alignment datum of each nucleic acid bin; and performing a correction operation on the initial number of sequences to obtain the number of nucleic acid sequences in the alignment datum of each nucleic acid bin.

In an exemplary embodiment, the initial number of sequences is the initial number of nucleic acid fragments in the chromosome sequencing datum and aligned to a specified nucleic acid bin, and the number of nucleic acid sequences is the corrected number of nucleic acid fragments in the chromosome sequencing datum and aligned to the specified nucleic acid bin.

In an optional embodiment, the correction operation includes at least one of effective base length correction, outlier correction, mappability correction or guanine-cytosine (GC)-content correction. A mappability value may be used for representing an alignment ability of the alignment tool to correctly align the chromosome sequencing datum to a nucleic acid bin in the human reference genome. The mappability correction refers to local polynomial regression fitting correction performed on the initial number of sequences in the alignment datum of the nucleic acid bin according to the mappability value. Since the initial number of sequences acquired from the alignment datum of the nucleic acid bin with a high GC content or a low GC content is less than the initial number of sequences acquired from the alignment datum of the nucleic acid bin with an intermediate GC content, the GC-content correction refers to normalization correction or local polynomial regression fitting correction performed on the initial number of sequences in the alignment datum of the nucleic acid bin according to the GC content of the alignment datum of the nucleic acid bin.

Such setting has the following advantage: different effective base lengths, different outliers, different mappability values and different GC contents are prevented from causing error interference to the sequencing depth of the chromosome, further improving the accuracy of the aneuploidy detection result.

In an optional embodiment, determining the sequencing depth sequence of the chromosome under test according to the sequencing depth of the chromosome under test and the sequencing depth of each preset chromosome includes: determining at least one reference sequencing depth ratio according to the sequencing depth of the chromosome under test and the sequencing depth of each preset chromosome, where each reference sequencing depth ratio is the ratio of the sequencing depth of the chromosome under test to a sequencing depth of one respective preset chromosome; and determining the sequencing depth sequence of the chromosome under test according to the at least one reference sequencing depth ratio.

In an optional embodiment, the sequencing depth parameter is the reference sequencing depth ratio. For example, the reference sequencing depth ratio of the chromosome under test i and the preset chromosome *j* may be represented as tᵢⱼ = Hᵢ/Hⱼ, where *i* ≠ *j,* Hᵢ denotes the sequencing depth of the chromosome under test i, and Hⱼ denotes the sequencing depth of the preset chromosome j. For example, a sequencing depth sequence T₁ of chromosome 1 may be represented as T₁ = [t₁₂, t₁₃, t₁₄, ..., t₁ⱼ].

In S130, according to the chromosome bin sequence and the sequencing depth sequence, a non-parametric test is performed so that the aneuploidy detection result of the chromosome under test in the nucleic acid sample under test is determined.

The aneuploidy of a chromosome refers to the loss or redundancy of the chromosome in the number of chromosomes relative to a normal disomy and is usually a trisomy or a monosomy.

In an exemplary embodiment, as can be known from the definition of the chromosome bin sequence and the definition of the sequencing depth sequence, the chromosome bin sequence of the chromosome under test is a fixed constant sequence; when the chromosome under test is a euploidy (disomy) in the nucleic acid sample under test, the distributions of the chromosome bin sequence and the sequencing depth sequence of the chromosome under test have no significant difference; when the chromosome under test is the aneuploidy in the nucleic acid sample under test, for example, if chromosome 21 is a trisomy, the sequencing depth of chromosome 21 becomes larger and thus the whole sequencing depth sequence T₂₁ becomes larger, if chromosome 21 is a monosomy, the sequencing depth of chromosome 21 becomes smaller and thus the whole sequencing depth sequence T₂₁ becomes smaller, and a change in the sequencing depth sequence causes a difference between the distributions of the chromosome bin sequence and the sequencing depth sequence of the chromosome under test.

The non-parametric test is used for determining whether the chromosome bin sequence and the sequencing depth sequence have a significant difference. In the presence of a significant difference, the aneuploidy detection result of the chromosome under test in the nucleic acid sample under test is the aneuploidy. In the presence of no significant difference, the aneuploidy detection result of the chromosome under test in the nucleic acid sample under test is the euploidy.

For example, the non-parametric test includes, but is not limited to, a chi-squared test, a K-S test, a Jonckheere-Terpstra test, a Mann-Whitney U test or a permutation test. The non-parametric test is not limited herein and may be customized according to the actual requirements.

Assuming that two or more chromosomes are aneuploidies in the nucleic acid sample under test, which is rare in reality, if the chromosome under test and a single preset chromosome are both aneuploidies in the nucleic acid sample under test, the overall change trend of the sequencing depth sequence may be eliminated. In this embodiment, multiple preset chromosomes are provided, that is, the sequencing depth sequence includes multiple sequencing depth parameters so that an effect of multiple aneuploid chromosomes in the nucleic acid sample under test on the overall change trend of the sequencing depth sequence can be avoided as much as possible, thereby improving the stability of the aneuploidy detection result of the chromosome and improving the accuracy of the aneuploidy detection result of the chromosome.

According to the technical solutions of this embodiment, the chromosome bin sequence built according to the human reference genome is used as a reference sequence of the chromosome under test, and the non-parametric test is performed according to the chromosome bin sequence and the sequencing depth sequence corresponding to the nucleic acid sample under test by using a correlation between a chromosome bin sequence and a sequencing depth sequence of a chromosome in nucleic acid data of euploidies so that the aneuploidy detection result of the chromosome under test in the nucleic acid sample under test is determined. The method has relatively high detection accuracy and solves the problem of dependence of the method for detecting chromosomal aneuploidy on indicator distribution in a normal sample so that a process of detecting chromosomal aneuploidy is no longer limited by a requirement for consistency between environmental parameters, and detection and maintenance costs of chromosomal aneuploidy are reduced.

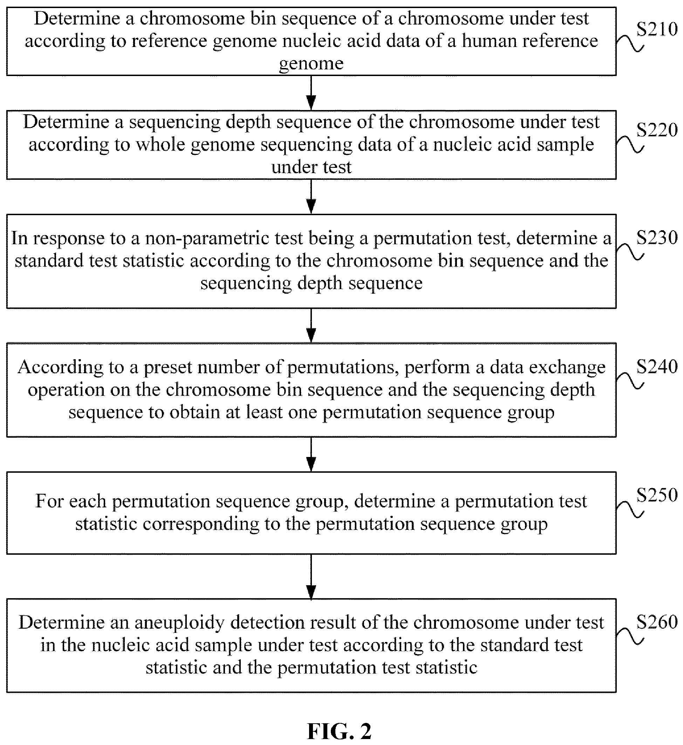

FIG. 2 is another flowchart of a method for detecting chromosomal aneuploidy according to an embodiment of the present invention. In this embodiment, that "according to the chromosome bin sequence and the sequencing depth sequence, the non-parametric test is performed so that the aneuploidy detection result of the chromosome under test in the nucleic acid sample under test is determined" in the preceding embodiment is further refined. As shown in FIG. 2, the method includes S210, S220, S230, S240, S250 and S260.

In S210, a chromosome bin sequence of a chromosome under test is determined according to reference genome nucleic acid data of a human reference genome.

S210 in this embodiment is the same as or similar to S110 shown in FIG. 1 in the preceding embodiment, and the details are not repeated in this embodiment.

In S220, a sequencing depth sequence of the chromosome under test is determined according to whole genome sequencing data of a nucleic acid sample under test.

In an optional embodiment, a sequencing depth parameter is a reference sequencing depth ratio, S220 in this embodiment is the same as or similar to S120 shown in FIG. 1 in the preceding embodiment, and the details are not repeated here.

In another optional embodiment, the sequencing depth parameter is a linear sequencing depth ratio. For example, the linear sequencing depth ratio is a linear proportional relationship between a sequencing depth of the chromosome under test in the nucleic acid sample under test and a sequencing depth of a preset chromosome in the nucleic acid sample under test.

In this embodiment, determining the sequencing depth sequence of the chromosome under test according to at least one reference sequencing depth ratio includes: in response to the sequencing depth parameter being the linear sequencing depth ratio, acquiring at least one sequence of sequencing depth ratios corresponding to at least one euploidy sample; building a matrix of sequencing depth ratios according to the at least one sequence of sequencing depth ratios; performing optimization according to the matrix of sequencing depth ratios and the chromosome bin sequence to obtain at least one linear fitting parameter corresponding to the chromosome under test; and performing a linear correction operation on the at least one reference sequencing depth ratio separately according to the at least one linear fitting parameter to obtain at least one linear sequencing depth ratio.

In an exemplary embodiment, the euploidy sample is used for representing a sample where at least the chromosome under test and at least one preset chromosome are euploidies. In this embodiment, each sequence of sequencing depth ratios corresponds to one respective euploidy sample and includes at least one standard sequencing depth ratio, and each standard sequencing depth ratio is the ratio of a sequencing depth of the chromosome under test in the euploidy sample to a sequencing depth of one respective preset chromosome in the euploidy sample.

The standard sequencing depth ratio in the sequence of sequencing depth ratios is acquired in a manner the same as or similar to a manner of acquiring the reference sequencing depth ratio in the preceding embodiment, and the details are not repeated in this embodiment.

For example, the matrix of sequencing depth ratios is an N×M matrix or an M×N matrix, where M denotes the number of euploidy samples and N denotes the number of preset chromosomes. For example, when the matrix of sequencing depth ratios is the N×M matrix, each matrix row of the matrix of sequencing depth ratios represents one sequence of sequencing depth ratios.

In an optional embodiment, after a linear depth ratio matrix is built according to the matrix of sequencing depth ratios, the method further includes: performing regularization on the matrix of sequencing depth ratios. Such setting has the following advantage: positive definiteness of the matrix of sequencing depth ratios can be ensured.

In an optional embodiment, constraints for the optimization include that an absolute value of a difference between the sequencing depth sequence and the chromosome bin sequence is minimum and that a slope parameter in each linear fitting parameter is greater than a preset positive threshold.

For example, the linear sequencing depth ratio of the chromosome under test i and the preset chromosome *j* may be represented as tactᵢⱼ = wᵢⱼ × tᵢⱼ + bᵢⱼ, where wᵢⱼ denotes a slope parameter corresponding to the chromosome under test *i* and the preset chromosome *j,* and bᵢⱼ denotes a constant parameter corresponding to the chromosome under test i and the preset chromosome *j*. Accordingly, a sum of |tactᵢⱼ-rᵢⱼ| is minimum and wᵢⱼ is greater than the preset positive threshold.

Under an ideal condition, a chromosome bin sequence of the euploidy sample is equal to a sequencing depth sequence of the euploidy sample including a reference sequencing depth ratio. However, since whole genome sequencing data are randomly and uniformly distributed, the chromosome bin sequence of the euploidy sample is positively correlated to the sequencing depth sequence of the euploidy sample including the reference sequencing depth ratio. In this embodiment, the linear correction is performed on the reference sequencing depth ratio according to the sequence of sequencing depth ratios of the euploidy sample, thereby improving the accuracy of the sequencing depth sequence and improving chromosomal aneuploidy detection performance such as sensitivity and specificity.

In S230, in response to the non-parametric test being a permutation test, a standard test statistic is determined according to the chromosome bin sequence and the sequencing depth sequence.

In this embodiment, the standard test statistic is a difference between a sequence mean of the chromosome bin sequence and a sequence mean of the sequencing depth sequence.

In S240, according to a preset number of permutations, a data exchange operation is performed on the chromosome bin sequence and the sequencing depth sequence so that at least one permutation sequence group is obtained.

For example, the preset number of permutations may be 50,000. The preset number of permutations is not limited herein and may be customized according to the actual requirements.

In this embodiment, each permutation sequence group includes a respective permuted chromosome bin sequence and a respective permuted sequencing depth sequence.

In S250, for each permutation sequence group, a permutation test statistic corresponding to the permutation sequence group is determined.

In this embodiment, the permutation test statistic is a difference between a sequence mean of the permuted chromosome bin sequence in the permutation sequence group and a sequence mean of the permuted sequencing depth sequence in the permutation sequence group.

In S260, the aneuploidy detection result of the chromosome under test in the nucleic acid sample under test is determined according to the standard test statistic and the permutation test statistic.

In an optional embodiment, that the aneuploidy detection result of the chromosome under test in the nucleic acid sample under test is determined according to the standard test statistic and at least one permutation test statistic includes: using a permutation test statistic greater than the standard test statistic among the at least one permutation test statistic as a target test statistic; using the ratio of a data volume of the target test statistic to the preset number of permutations as a test probability value; in response to the test probability value being less than a significance level, determining the aneuploidy detection result of the chromosome under test in the nucleic acid sample under test to be an aneuploidy; and in response to the test probability value being greater than or equal to the significance level, determining the aneuploidy detection result of the chromosome under test in the nucleic acid sample under test to be a euploidy.

For example, the significance level may be 0.01 or 0.001. The significance level is not limited herein and may be customized according to the actual requirements.

In an exemplary embodiment, it is assumed that a null hypothesis H0 is established that the distributions of the chromosome bin sequence and the sequencing depth sequence have no difference, that is, the chromosome under test is the euploidy in the nucleic acid sample under test; and it is assumed that an alternative hypothesis H1 is established that the distributions of the chromosome bin sequence and the sequencing depth sequence have a difference, that is, the chromosome under test is the aneuploidy in the nucleic acid sample under test. If the test probability value P is less than the significance level, the null hypothesis H0 is rejected, that is, the aneuploidy detection result of the chromosome under test in the nucleic acid sample under test is determined to be the aneuploidy. If the test probability value P is greater than or equal to the significance level, the null hypothesis H0 is accepted, that is, the aneuploidy detection result of the chromosome under test in the nucleic acid sample under test is determined to be the euploidy.

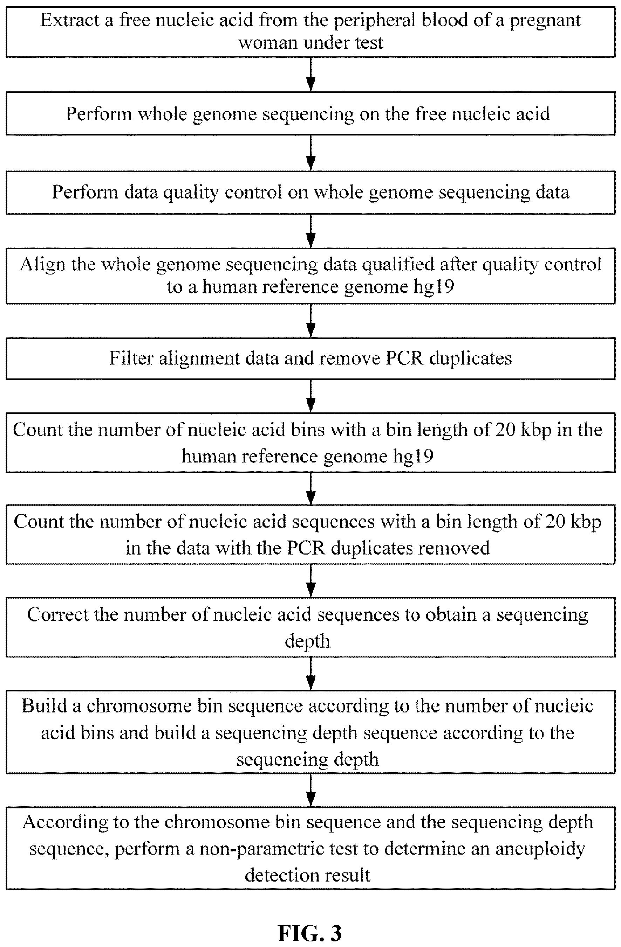

FIG. 3 is a flowchart of an example of a method for detecting chromosomal aneuploidy according to an embodiment of the present invention. The peripheral blood of a pregnant woman under test is used as a nucleic acid sample under test, a free nucleic acid is extracted from the peripheral blood of the pregnant woman under test, and whole genome sequencing is performed on the free nucleic acid so that whole genome sequencing data are obtained. Data quality control is performed on the whole genome sequencing data. For example, a quality control tool used for the data quality control may be a fastp tool, a Trimmomatic tool or a FastQC tool. The quality control tool used for quality control is not limited herein and may be customized according to the actual requirements. The whole genome sequencing data qualified after quality control are aligned to reference genome nucleic acid data of a human reference genome hg19, the obtained alignment data are filtered, and PCR duplicates are removed.

The number of nucleic acid bins each having a bin length of 20 kbp in the reference genome nucleic acid data of the human reference genome hg19 is counted, the number of nucleic acid sequences in a bin length of 20 kbp is counted in the alignment data with the PCR duplicates removed and corrected, and a sequencing depth is determined according to the number of nucleic acid sequences corresponding to multiple bins each having a bin length of 20 kbp.

A chromosome bin sequence is built according to the number of nucleic acid bins of each of multiple chromosomes, a sequencing depth sequence is built according to the sequencing depth of each of the multiple chromosomes, and according to the chromosome bin sequence and the sequencing depth sequence, a non-parametric test is performed so that an aneuploidy detection result of the peripheral blood of the pregnant woman under test is determined. The aneuploidy detection result of the peripheral blood of the pregnant woman under test includes a respective aneuploidy detection result of at least one chromosome.

According to the technical solutions of this embodiment, according to the chromosome bin sequence and the sequencing depth sequence, a permutation test is performed so that an aneuploidy detection result of a chromosome under test in the nucleic acid sample under test is obtained, thereby solving the problem of the non-parametric test in the method for detecting chromosomal aneuploidy and ensuring the accuracy of the aneuploidy detection result of the chromosome.

The following description is provided in conjunction with embodiments.

### Embodiment one

Whole genome sequencing data of 63 euploidy samples are used for obtaining standard sequencing depth ratios through the preceding steps such as bin counting, alignment, sequencing depth determination and sequencing depth correction. Then, a matrix of sequencing depth ratios is built according to the standard sequencing depth ratios, and optimization is performed according to a chromosome bin sequence and the matrix of sequencing depth ratios to obtain a linear fitting parameter.

In embodiment one, the above 63 euploidy samples are each used as a nucleic acid sample under test and checked by the method for detecting chromosomal aneuploidy according to the embodiments of the present invention.

Table 1 below shows test probability values P of each of chromosome 1 to chromosome 6 corresponding to the 63 euploidy samples according to embodiment one of the present invention.

| No. | T1_pv | T2_pv | T3_pv | T4_pv | T5_pv | T6_pv |
|---|---|---|---|---|---|---|
| A07 | 0.483 | 0.448 | 0.493 | 0.473 | 0.580 | 0.453 |
| A08 | 0.503 | 0.400 | 0.514 | 0.490 | 0.511 | 0.536 |
| A09 | 0.560 | 0.460 | 0.502 | 0.427 | 0.264 | 0.488 |
| A10 | 0.646 | 0.572 | 0.538 | 0.440 | 0.474 | 0.533 |
| A11 | 0.483 | 0.457 | 0.458 | 0.348 | 0.466 | 0.474 |
| A12 | 0.432 | 0.480 | 0.462 | 0.418 | 0.461 | 0.488 |
| B07 | 0.519 | 0.455 | 0.516 | 0.501 | 0.555 | 0.483 |
| B08 | 0.518 | 0.462 | 0.530 | 0.454 | 0.500 | 0.459 |
| B10 | 0.474 | 0.429 | 0.535 | 0.517 | 0.502 | 0.539 |
| B11 | 0.527 | 0.344 | 0.487 | 0.468 | 0.489 | 0.499 |
| B12 | 0.388 | 0.390 | 0.449 | 0.443 | 0.425 | 0.396 |
| C07 | 0.503 | 0.430 | 0.496 | 0.274 | 0.485 | 0.403 |
| C08 | 0.611 | 0.433 | 0.469 | 0.463 | 0.509 | 0.491 |
| C09 | 0.492 | 0.422 | 0.485 | 0.439 | 0.486 | 0.499 |
| C11 | 0.631 | 0.562 | 0.601 | 0.683 | 0.532 | 0.555 |
| C12 | 0.391 | 0.391 | 0.475 | 0.270 | 0.437 | 0.351 |
| D07 | 0.493 | 0.461 | 0.455 | 0.430 | 0.455 | 0.418 |
| D08 | 0.609 | 0.501 | 0.516 | 0.621 | 0.636 | 0.717 |
| D09 | 0.458 | 0.528 | 0.549 | 0.492 | 0.532 | 0.489 |
| D10 | 0.538 | 0.467 | 0.517 | 0.468 | 0.467 | 0.546 |
| D11 | 0.782 | 0.580 | 0.708 | 0.499 | 0.556 | 0.688 |
| D12 | 0.514 | 0.477 | 0.504 | 0.481 | 0.491 | 0.596 |
| E07 | 0.469 | 0.305 | 0.474 | 0.492 | 0.475 | 0.427 |
| E08 | 0.546 | 0.494 | 0.486 | 0.613 | 0.612 | 0.569 |
| E09 | 0.501 | 0.398 | 0.485 | 0.364 | 0.496 | 0.463 |
| E10 | 0.473 | 0.427 | 0.446 | 0.449 | 0.489 | 0.450 |
| E11 | 0.709 | 0.524 | 0.650 | 0.448 | 0.505 | 0.666 |
| E12 | 0.592 | 0.453 | 0.500 | 0.429 | 0.460 | 0.607 |
| F08 | 0.590 | 0.500 | 0.640 | 0.487 | 0.500 | 0.612 |
| F09 | 0.433 | 0.315 | 0.473 | 0.495 | 0.545 | 0.462 |
| F10 | 0.453 | 0.483 | 0.488 | 0.479 | 0.433 | 0.387 |
| F11 | 0.748 | 0.576 | 0.678 | 0.499 | 0.570 | 0.687 |
| F12 | 0.524 | 0.456 | 0.461 | 0.483 | 0.512 | 0.482 |
| G07 | 0.428 | 0.348 | 0.362 | 0.483 | 0.495 | 0.394 |
| G08 | 0.683 | 0.503 | 0.557 | 0.592 | 0.488 | 0.606 |
| G09 | 0.547 | 0.394 | 0.463 | 0.440 | 0.498 | 0.532 |
| G10 | 0.503 | 0.425 | 0.503 | 0.386 | 0.498 | 0.469 |
| G12 | 0.512 | 0.467 | 0.498 | 0.513 | 0.498 | 0.493 |
| H07 | 0.438 | 0.358 | 0.480 | 0.477 | 0.489 | 0.474 |
| H08 | 0.500 | 0.483 | 0.511 | 0.474 | 0.526 | 0.517 |
| H09 | 0.617 | 0.450 | 0.384 | 0.304 | 0.423 | 0.506 |
| H10 | 0.505 | 0.476 | 0.510 | 0.480 | 0.483 | 0.497 |
| H11 | 0.503 | 0.499 | 0.528 | 0.464 | 0.485 | 0.575 |
| H12 | 0.486 | 0.470 | 0.494 | 0.477 | 0.484 | 0.476 |
| XY10 | 0.443 | 0.349 | 0.426 | 0.490 | 0.521 | 0.454 |
| XY11 | 0.714 | 0.414 | 0.502 | 0.285 | 0.452 | 0.493 |
| XY13 | 0.501 | 0.474 | 0.524 | 0.482 | 0.490 | 0.527 |
| XY14 | 0.516 | 0.478 | 0.505 | 0.500 | 0.499 | 0.557 |
| XY15 | 0.847 | 0.557 | 0.809 | 0.492 | 0.523 | 0.686 |
| XY16 | 0.585 | 0.479 | 0.505 | 0.490 | 0.502 | 0.495 |
| XY17 | 0.635 | 0.610 | 0.640 | 0.616 | 0.577 | 0.682 |
| XY18 | 0.755 | 0.466 | 0.727 | 0.482 | 0.485 | 0.525 |
| XY19 | 0.538 | 0.475 | 0.594 | 0.458 | 0.513 | 0.495 |
| XY1 | 0.800 | 0.418 | 0.674 | 0.494 | 0.649 | 0.707 |
| XY20 | 0.577 | 0.432 | 0.688 | 0.736 | 0.712 | 0.713 |
| XY2 | 0.653 | 0.490 | 0.505 | 0.483 | 0.605 | 0.675 |
| XY3 | 0.649 | 0.465 | 0.532 | 0.500 | 0.656 | 0.616 |
| XY4 | 0.519 | 0.475 | 0.485 | 0.453 | 0.492 | 0.500 |
| XY5 | 0.578 | 0.470 | 0.542 | 0.478 | 0.490 | 0.488 |
| XY6 | 0.649 | 0.634 | 0.777 | 0.690 | 0.761 | 0.778 |
| XY7 | 0.742 | 0.524 | 0.601 | 0.722 | 0.621 | 0.841 |
| XY8 | 0.579 | 0.459 | 0.591 | 0.480 | 0.568 | 0.538 |
| XY9 | 0.615 | 0.459 | 0.575 | 0.552 | 0.493 | 0.491 |

Table 2 below shows test probability values P of each of chromosome 7 to chromosome 12 corresponding to the 63 euploidy samples according to embodiment one of the present invention.

| No. | T7_pv | T8_pv | T9_pv | T10_pv | T11_pv | T12_pv |
|---|---|---|---|---|---|---|
| A07 | 0.645 | 0.424 | 0.428 | 0.400 | 0.466 | 0.503 |
| A08 | 0.694 | 0.493 | 0.503 | 0.467 | 0.545 | 0.506 |
| A09 | 0.578 | 0.298 | 0.497 | 0.450 | 0.226 | 0.500 |
| A10 | 0.786 | 0.413 | 0.414 | 0.515 | 0.535 | 0.514 |
| A11 | 0.604 | 0.395 | 0.433 | 0.368 | 0.426 | 0.480 |
| A12 | 0.725 | 0.443 | 0.411 | 0.463 | 0.512 | 0.346 |
| B07 | 0.803 | 0.480 | 0.465 | 0.454 | 0.484 | 0.481 |
| B08 | 0.723 | 0.457 | 0.477 | 0.488 | 0.455 | 0.494 |
| B10 | 0.761 | 0.486 | 0.467 | 0.395 | 0.534 | 0.499 |
| B11 | 0.667 | 0.469 | 0.412 | 0.277 | 0.441 | 0.474 |
| B12 | 0.781 | 0.416 | 0.461 | 0.394 | 0.455 | 0.448 |
| C07 | 0.498 | 0.293 | 0.507 | 0.482 | 0.406 | 0.405 |
| C08 | 0.768 | 0.448 | 0.472 | 0.308 | 0.466 | 0.460 |
| C09 | 0.669 | 0.498 | 0.443 | 0.370 | 0.424 | 0.539 |
| C11 | 0.891 | 0.482 | 0.523 | 0.571 | 0.481 | 0.491 |
| C12 | 0.550 | 0.479 | 0.432 | 0.449 | 0.402 | 0.478 |
| D07 | 0.607 | 0.267 | 0.505 | 0.493 | 0.329 | 0.511 |
| D08 | 0.909 | 0.493 | 0.785 | 0.445 | 0.543 | 0.530 |
| D09 | 0.855 | 0.559 | 0.397 | 0.463 | 0.507 | 0.536 |
| D10 | 0.681 | 0.380 | 0.509 | 0.322 | 0.466 | 0.491 |
| D11 | 0.835 | 0.493 | 0.496 | 0.521 | 0.474 | 0.518 |
| D12 | 0.612 | 0.476 | 0.467 | 0.461 | 0.486 | 0.493 |
| E07 | 0.690 | 0.459 | 0.415 | 0.371 | 0.455 | 0.421 |
| E08 | 0.887 | 0.470 | 0.501 | 0.487 | 0.425 | 0.550 |
| E09 | 0.562 | 0.384 | 0.523 | 0.463 | 0.323 | 0.480 |
| E10 | 0.686 | 0.474 | 0.460 | 0.238 | 0.446 | 0.488 |
| E11 | 0.669 | 0.410 | 0.490 | 0.424 | 0.529 | 0.578 |
| E12 | 0.674 | 0.362 | 0.398 | 0.487 | 0.405 | 0.531 |
| F08 | 0.917 | 0.461 | 0.481 | 0.500 | 0.462 | 0.490 |
| F09 | 0.814 | 0.561 | 0.378 | 0.403 | 0.475 | 0.504 |
| F10 | 0.622 | 0.438 | 0.528 | 0.432 | 0.425 | 0.523 |
| F11 | 0.804 | 0.417 | 0.488 | 0.468 | 0.543 | 0.498 |
| F12 | 0.713 | 0.480 | 0.485 | 0.493 | 0.486 | 0.514 |
| G07 | 0.681 | 0.455 | 0.383 | 0.173 | 0.209 | 0.474 |
| G08 | 0.787 | 0.471 | 0.474 | 0.484 | 0.205 | 0.491 |
| G09 | 0.795 | 0.481 | 0.476 | 0.524 | 0.330 | 0.430 |
| G10 | 0.643 | 0.470 | 0.476 | 0.487 | 0.426 | 0.471 |
| G12 | 0.628 | 0.477 | 0.488 | 0.460 | 0.415 | 0.498 |
| H07 | 0.665 | 0.465 | 0.469 | 0.450 | 0.475 | 0.458 |
| H08 | 0.583 | 0.464 | 0.491 | 0.453 | 0.449 | 0.505 |
| H09 | 0.491 | 0.484 | 0.481 | 0.403 | 0.487 | 0.486 |
| H10 | 0.808 | 0.498 | 0.492 | 0.496 | 0.492 | 0.423 |
| H11 | 0.882 | 0.422 | 0.453 | 0.568 | 0.436 | 0.524 |
| H12 | 0.690 | 0.494 | 0.525 | 0.472 | 0.468 | 0.494 |
| XY10 | 0.582 | 0.402 | 0.460 | 0.430 | 0.447 | 0.470 |
| XY11 | 0.519 | 0.433 | 0.453 | 0.474 | 0.443 | 0.486 |
| XY13 | 0.553 | 0.468 | 0.465 | 0.384 | 0.422 | 0.493 |
| XY14 | 0.616 | 0.326 | 0.422 | 0.308 | 0.366 | 0.572 |
| XY15 | 0.721 | 0.421 | 0.419 | 0.489 | 0.429 | 0.513 |
| XY16 | 0.600 | 0.450 | 0.378 | 0.528 | 0.539 | 0.588 |
| XY17 | 0.867 | 0.521 | 0.457 | 0.464 | 0.466 | 0.532 |
| XY18 | 0.774 | 0.340 | 0.484 | 0.468 | 0.496 | 0.534 |
| XY19 | 0.567 | 0.447 | 0.484 | 0.387 | 0.472 | 0.633 |
| XY1 | 0.825 | 0.493 | 0.497 | 0.486 | 0.507 | 0.572 |
| XY20 | 0.911 | 0.610 | 0.421 | 0.432 | 0.596 | 0.461 |
| XY2 | 0.854 | 0.276 | 0.342 | 0.418 | 0.440 | 0.739 |
| XY3 | 0.806 | 0.473 | 0.424 | 0.258 | 0.586 | 0.704 |
| XY4 | 0.495 | 0.312 | 0.485 | 0.337 | 0.462 | 0.505 |
| XY5 | 0.700 | 0.412 | 0.453 | 0.320 | 0.470 | 0.532 |
| XY6 | 0.895 | 0.549 | 0.287 | 0.492 | 0.542 | 0.630 |
| XY7 | 0.843 | 0.259 | 0.435 | 0.608 | 0.304 | 0.516 |
| XY8 | 0.667 | 0.453 | 0.481 | 0.323 | 0.507 | 0.515 |
| XY9 | 0.761 | 0.457 | 0.489 | 0.460 | 0.473 | 0.500 |

Table 3 below shows test probability values P of each of chromosome 13 to chromosome 17 corresponding to the 63 euploidy samples according to embodiment one of the present invention.

| No. | T13_pv | T14_pv | T15_pv | T16_pv | T17_pv |
|---|---|---|---|---|---|
| A07 | 0.470 | 0.449 | 0.467 | 0.761 | 0.235 |
| A08 | 0.460 | 0.387 | 0.584 | 0.784 | 0.388 |
| A09 | 0.431 | 0.474 | 0.523 | 0.357 | 0.499 |
| A10 | 0.514 | 0.543 | 0.497 | 0.584 | 0.457 |
| A11 | 0.493 | 0.477 | 0.496 | 0.552 | 0.672 |
| A12 | 0.467 | 0.424 | 0.495 | 0.667 | 0.202 |
| B07 | 0.457 | 0.466 | 0.451 | 0.778 | 0.456 |
| B08 | 0.402 | 0.452 | 0.505 | 0.702 | 0.447 |
| B10 | 0.493 | 0.536 | 0.507 | 0.721 | 0.268 |
| B11 | 0.483 | 0.471 | 0.491 | 0.613 | 0.494 |
| B12 | 0.458 | 0.406 | 0.276 | 0.900 | 0.509 |
| C07 | 0.144 | 0.426 | 0.501 | 0.509 | 0.490 |
| C08 | 0.446 | 0.413 | 0.562 | 0.825 | 0.446 |
| C09 | 0.455 | 0.568 | 0.507 | 0.562 | 0.496 |
| C11 | 0.459 | 0.500 | 0.919 | 0.502 | 0.418 |
| C12 | 0.471 | 0.442 | 0.081 | 0.765 | 0.475 |
| D07 | 0.434 | 0.482 | 0.524 | 0.533 | 0.500 |
| D08 | 0.518 | 0.453 | 0.531 | 0.833 | 0.504 |
| D09 | 0.505 | 0.442 | 0.493 | 0.608 | 0.300 |
| D10 | 0.485 | 0.484 | 0.467 | 0.557 | 0.501 |
| D11 | 0.484 | 0.456 | 0.504 | 0.490 | 0.461 |
| D12 | 0.490 | 0.192 | 0.691 | 0.623 | 0.506 |
| E07 | 0.480 | 0.519 | 0.473 | 0.761 | 0.462 |
| E08 | 0.485 | 0.513 | 0.499 | 0.814 | 0.324 |
| E09 | 0.316 | 0.318 | 0.549 | 0.491 | 0.498 |
| E10 | 0.494 | 0.422 | 0.483 | 0.510 | 0.483 |
| E11 | 0.347 | 0.411 | 0.467 | 0.466 | 0.524 |
| E12 | 0.456 | 0.466 | 0.543 | 0.732 | 0.477 |
| F08 | 0.477 | 0.502 | 0.517 | 0.502 | 0.402 |
| F09 | 0.468 | 0.426 | 0.458 | 0.764 | 0.327 |
| F10 | 0.417 | 0.464 | 0.498 | 0.863 | 0.425 |
| F11 | 0.469 | 0.376 | 0.209 | 0.471 | 0.517 |
| F12 | 0.465 | 0.451 | 0.498 | 0.731 | 0.514 |
| G07 | 0.469 | 0.509 | 0.474 | 0.941 | 0.479 |
| G08 | 0.497 | 0.426 | 0.601 | 0.531 | 0.366 |
| G09 | 0.465 | 0.444 | 0.688 | 0.564 | 0.375 |
| G10 | 0.442 | 0.467 | 0.500 | 0.515 | 0.495 |
| G12 | 0.462 | 0.455 | 0.431 | 0.769 | 0.432 |
| H07 | 0.386 | 0.481 | 0.483 | 0.867 | 0.348 |
| H08 | 0.488 | 0.471 | 0.489 | 0.757 | 0.477 |
| H09 | 0.413 | 0.146 | 0.591 | 0.812 | 0.513 |
| H10 | 0.371 | 0.472 | 0.519 | 0.641 | 0.457 |
| H11 | 0.490 | 0.429 | 0.649 | 0.521 | 0.311 |
| H12 | 0.422 | 0.488 | 0.508 | 0.785 | 0.524 |
| XY10 | 0.497 | 0.494 | 0.475 | 0.782 | 0.309 |
| XY11 | 0.465 | 0.484 | 0.680 | 0.386 | 0.637 |
| XY13 | 0.495 | 0.489 | 0.501 | 0.509 | 0.592 |
| XY14 | 0.491 | 0.487 | 0.502 | 0.456 | 0.514 |
| XY15 | 0.460 | 0.573 | 0.607 | 0.364 | 0.480 |
| XY16 | 0.448 | 0.473 | 0.623 | 0.574 | 0.549 |
| XY17 | 0.681 | 0.490 | 0.825 | 0.440 | 0.296 |
| XY18 | 0.411 | 0.557 | 0.685 | 0.467 | 0.458 |
| XY19 | 0.555 | 0.512 | 0.545 | 0.657 | 0.504 |
| XY1 | 0.541 | 0.475 | 0.812 | 0.072 | 0.526 |
| XY20 | 0.796 | 0.582 | 0.362 | 0.727 | 0.262 |
| XY2 | 0.477 | 0.569 | 0.637 | 0.364 | 0.447 |
| XY3 | 0.539 | 0.458 | 0.467 | 0.579 | 0.375 |
| XY4 | 0.498 | 0.530 | 0.712 | 0.379 | 0.490 |
| XY5 | 0.545 | 0.472 | 0.488 | 0.633 | 0.501 |
| XY6 | 0.772 | 0.542 | 0.496 | 0.530 | 0.316 |
| XY7 | 0.938 | 0.622 | 0.508 | 0.071 | 0.394 |
| XY8 | 0.503 | 0.545 | 0.635 | 0.689 | 0.464 |
| XY9 | 0.550 | 0.366 | 0.564 | 0.504 | 0.460 |

Table 4 below shows test probability values P of each of chromosome 18 to chromosome 22 corresponding to the 63 euploidy samples according to embodiment one of the present invention.

| No. | T18_pv | T19_pv | T20_pv | T21_pv | T22_pv |
|---|---|---|---|---|---|
| A07 | 0.494 | 1.000 | 0.546 | 0.545 | 0.787 |
| A08 | 0.500 | 1.000 | 0.495 | 0.501 | 0.570 |
| A09 | 0.501 | 0.991 | 0.795 | 0.577 | 0.988 |
| A10 | 0.465 | 0.983 | 0.538 | 0.545 | 0.816 |
| A11 | 0.358 | 1.000 | 0.622 | 0.558 | 0.508 |
| A12 | 0.537 | 1.000 | 0.612 | 0.705 | 0.707 |
| B07 | 0.505 | 1.000 | 0.716 | 0.488 | 0.497 |
| B08 | 0.500 | 1.000 | 0.527 | 0.580 | 0.506 |
| B10 | 0.552 | 1.000 | 0.501 | 0.482 | 0.559 |
| B11 | 0.401 | 1.000 | 0.525 | 0.605 | 0.765 |
| B12 | 0.486 | 1.000 | 0.838 | 0.541 | 0.467 |
| C07 | 0.489 | 1.000 | 0.818 | 0.618 | 0.920 |
| C08 | 0.336 | 1.000 | 0.401 | 0.775 | 0.502 |
| C09 | 0.488 | 1.000 | 0.497 | 0.484 | 0.737 |
| C11 | 0.586 | 0.872 | 0.669 | 0.402 | 0.838 |
| C12 | 0.443 | 1.000 | 0.517 | 0.650 | 0.651 |
| D07 | 0.654 | 1.000 | 0.816 | 0.577 | 0.513 |
| D08 | 0.467 | 0.998 | 0.500 | 0.238 | 0.685 |
| D09 | 0.484 | 1.000 | 0.541 | 0.635 | 0.550 |
| D10 | 0.392 | 1.000 | 0.525 | 0.537 | 0.581 |
| D11 | 0.450 | 1.000 | 0.517 | 0.479 | 0.853 |
| D12 | 0.494 | 1.000 | 0.610 | 0.495 | 0.857 |
| E07 | 0.493 | 1.000 | 0.499 | 0.584 | 0.487 |
| E08 | 0.455 | 1.000 | 0.576 | 0.476 | 0.470 |
| E09 | 0.480 | 1.000 | 0.534 | 0.590 | 0.714 |
| E10 | 0.473 | 1.000 | 0.498 | 0.588 | 0.600 |
| E11 | 0.375 | 1.000 | 0.597 | 0.618 | 0.660 |
| E12 | 0.437 | 1.000 | 0.748 | 0.544 | 0.495 |
| F08 | 0.489 | 1.000 | 0.501 | 0.496 | 0.623 |
| F09 | 0.599 | 1.000 | 0.629 | 0.618 | 0.550 |
| F10 | 0.481 | 1.000 | 0.836 | 0.595 | 0.500 |
| F11 | 0.490 | 1.000 | 0.607 | 0.483 | 0.698 |
| F12 | 0.074 | 1.000 | 0.489 | 0.501 | 0.736 |
| G07 | 0.402 | 1.000 | 0.650 | 0.535 | 0.553 |
| G08 | 0.455 | 1.000 | 0.464 | 0.521 | 0.784 |
| G09 | 0.520 | 0.989 | 0.507 | 0.701 | 0.883 |
| G10 | 0.497 | 1.000 | 0.497 | 0.588 | 0.709 |
| G12 | 0.497 | 1.000 | 0.579 | 0.495 | 0.646 |
| H07 | 0.508 | 1.000 | 0.626 | 0.503 | 0.566 |
| H08 | 0.446 | 1.000 | 0.635 | 0.539 | 0.577 |
| H09 | 0.548 | 0.999 | 0.676 | 0.628 | 0.783 |
| H10 | 0.514 | 1.000 | 0.652 | 0.489 | 0.715 |
| H11 | 0.462 | 0.935 | 0.640 | 0.797 | 0.692 |
| H12 | 0.449 | 1.000 | 0.552 | 0.399 | 0.509 |
| XY10 | 0.521 | 1.000 | 0.504 | 0.631 | 0.519 |
| XY11 | 0.316 | 1.000 | 0.619 | 0.768 | 0.603 |
| XY13 | 0.437 | 1.000 | 0.503 | 0.506 | 0.735 |
| XY14 | 0.323 | 1.000 | 0.497 | 0.520 | 0.547 |
| XY15 | 0.173 | 0.843 | 0.506 | 0.797 | 0.839 |
| XY16 | 0.437 | 1.000 | 0.479 | 0.499 | 0.834 |
| XY17 | 0.504 | 0.819 | 0.609 | 0.581 | 0.674 |
| XY18 | 0.346 | 1.000 | 0.503 | 0.615 | 0.544 |
| XY19 | 0.280 | 1.000 | 0.502 | 0.477 | 0.634 |
| XY1 | 0.476 | 0.571 | 0.497 | 0.507 | 0.953 |
| XY20 | 0.502 | 1.000 | 0.331 | 0.769 | 0.012 |
| XY2 | 0.351 | 1.000 | 0.463 | 0.812 | 0.551 |
| XY3 | 0.426 | 1.000 | 0.443 | 0.523 | 0.494 |
| XY4 | 0.337 | 1.000 | 0.499 | 0.502 | 0.978 |
| XY5 | 0.422 | 1.000 | 0.494 | 0.485 | 0.624 |
| XY6 | 0.495 | 0.997 | 0.527 | 0.676 | 0.327 |
| XY7 | 0.558 | 0.822 | 0.179 | 0.892 | 0.568 |
| XY8 | 0.327 | 1.000 | 0.513 | 0.561 | 0.492 |
| XY9 | 0.526 | 1.000 | 0.467 | 0.523 | 0.574 |

In the above Table 1 to Table 4, the leftmost column represents sample numbers of the euploidy samples, and the other columns represent test probability values P of different human chromosomes corresponding to the 63 euploidy samples. For example, in "T1_pv", "T1" represents chromosome 1 and "pv" represents a test probability value P.

The check results in the above Table 1 to Table 4 show that the test probability value P of any human chromosome corresponding to each euploidy sample is greater than a significance level of 0.01, indicating that any human chromosome is a euploidy in each euploidy sample.

### Embodiment two

In the National Standard and Reference Material Catalogue of *In Vitro* Diagnostic Reagents for Registration and Testing (phase XI) published by the National Institutes for Food and Drug Control, the national reference materials of fetal chromosomal aneuploidy abnormality (T21, T18 and T13) in peripheral blood for next-generation sequencing (Variety 360008) is one of the important references for testing reagents and detection methods. Therefore, whole genome sequencing data measured by using the national reference materials are used as whole genome sequencing data of a nucleic acid sample under test to evaluate the detection performance of the preceding method for detecting chromosomal aneuploidy.

In the national reference materials of fetal chromosomal aneuploidy abnormality (T21, T18 and T13) in peripheral blood for next-generation sequencing, sample types of the national reference materials are recorded, where each sample type includes a number of a sample, a positive chromosome in the sample, a number of the positive chromosome and a preset concentration of the positive chromosome.

Table 5 below shows test probability values P of each of chromosome 1 to chromosome 6 corresponding to 93 national reference materials according to embodiment two of the present invention.

| National reference | chr1_pv | chr2_pv | chr3_pv | chr4_pv | chr5_pv | chr6_pv |
|---|---|---|---|---|---|---|
| 1-T21-1-10% | 0.77944 | 0.74077 | 0.76142 | 0.76768 | 0.82766 | 0.70411 |
| 2-T21-2-10% | 0.74053 | 0.7517 | 0.75166 | 0.75156 | 0.80894 | 0.75206 |
| 3-T21-3-10% | 0.7633 | 0.73829 | 0.75804 | 0.74493 | 0.73059 | 0.75378 |
| 4-T21-4-10% | 0.75976 | 0.75546 | 0.72781 | 0.7552 | 0.78636 | 0.74325 |
| 5-T21-5-10% | 0.76464 | 0.73785 | 0.74297 | 0.74833 | 0.74841 | 0.73877 |
| 6-T21-6-10% | 0.77664 | 2.00E-05 | 0.75966 | 0.73961 | 0.76536 | 0.7647 |
| 7-T18-1-10% | 0.48653 | 0.59033 | 0.72235 | 0.68743 | 0.69307 | 0.46887 |
| 8-T18-2-10% | 0.56065 | 0.46777 | 0.48877 | 0.43123 | 0.49209 | 0.44611 |
| 9-T18-3-10% | 0.67705 | 0.51877 | 0.57957 | 0.54737 | 0.52345 | 0.57247 |
| 10-T13-1-10% | 0.51313 | 0.49471 | 0.47785 | 0.49485 | 0.55219 | 0.49959 |
| 11-T13-2-10% | 0.61785 | 0.59151 | 0.63843 | 0.74503 | 0.76014 | 0.62725 |
| 12-T13-3-10% | 0.57255 | 0.56057 | 0.47807 | 0.55697 | 0.58569 | 0.51155 |
| 13-T21-1-5% | 0.60495 | 0.61985 | 0.70511 | 0.58931 | 0.67011 | 0.74545 |
| 14-T21-2-5% | 0.58997 | 0.57097 | 0.65039 | 0.65535 | 0.51341 | 0.68173 |
| 15-T21-3-5% | 0.83658 | 0.65671 | 0.67357 | 0.65983 | 0.69657 | 0.71799 |
| 16-T21-4-5% | 0.75714 | 0.60617 | 0.72293 | 0.80584 | 0.67629 | 0.72765 |
| 17-T21-5-5% | 0.76954 | 0.7506 | 0.56509 | 0.76954 | 0.75138 | 0.65411 |
| 18-T21-6-5% | 0.74331 | 2.00E-05 | 0.69445 | 0.72049 | 0.66805 | 0.79954 |
| 19-T18-1-5% | 0.74053 | 0.64661 | 0.73459 | 0.66539 | 0.62687 | 0.61009 |
| 20-T18-2-5% | 0.64665 | 0.51585 | 0.53741 | 0.65033 | 0.57709 | 0.67045 |
| 21-T18-3-5% | 0.49963 | 0.49027 | 0.50845 | 0.62201 | 0.58755 | 0.54933 |
| 22-T13-1-5% | 0.49749 | 0.47651 | 0.49857 | 0.49601 | 0.48145 | 0.42765 |
| 23-T13-2-5% | 0.53613 | 0.50435 | 0.56191 | 0.56925 | 0.55719 | 0.49705 |
| 24-T13-3-5% | 0.59567 | 0.48811 | 0.56281 | 0.79218 | 0.62903 | 0.57807 |
| 25-T21-1-3.5% | 0.67911 | 0.63401 | 0.73681 | 0.68957 | 0.70383 | 0.7832 |
| 26-T21-2-3.5% | 0.83494 | 0.69413 | 0.73613 | 0.69555 | 0.73165 | 0.61353 |
| 27-T21-3-3.5% | 0.72929 | 0.75598 | 0.61967 | 0.8572 | 0.78 | 0.71159 |
| 28-T21-4-3.5% | 0.83612 | 0.81022 | 0.71533 | 0.8708 | 0.64911 | 0.86554 |
| 29-T21-5-3.5% | 0.53793 | 0.59889 | 0.72299 | 0.67147 | 0.54943 | 0.51015 |
| 30-T21-6-3.5% | 0.7851 | 2.00E-05 | 0.55983 | 0.72483 | 0.71249 | 0.61597 |
| 31-T18-1-3.5% | 0.73827 | 0.70873 | 0.52549 | 0.79392 | 0.68867 | 0.75254 |
| 32-T18-2-3.5% | 0.59097 | 0.48239 | 0.52483 | 0.59245 | 0.57339 | 0.49185 |
| 33-T18-3-3.5% | 0.48805 | 0.33221 | 0.50137 | 0.49303 | 0.49679 | 0.43303 |
| 34-T13-1-3.5% | 0.29557 | 0.38227 | 0.25579 | 0.52825 | 0.51209 | 0.50207 |
| 35-T13-2-3.5% | 0.55881 | 0.49509 | 0.49969 | 0.49309 | 0.47151 | 0.50047 |
| 36-T13-3-3.5% | 0.53495 | 0.42175 | 0.47989 | 0.47037 | 0.57761 | 0.43803 |
| 37-T21-1-2.5% | 0.49261 | 0.48825 | 0.50347 | 0.57491 | 0.49373 | 0.59253 |
| 38-T21-2-2.5% | 0.52221 | 0.48361 | 0.53089 | 0.49913 | 0.59755 | 0.48309 |
| 39-T21-3-2.5% | 0.50103 | 0.48881 | 0.53217 | 0.38419 | 0.49779 | 0.49163 |
| 40-T21-4-2.5% | 0.63725 | 0.48763 | 0.58595 | 0.47715 | 0.52739 | 0.57713 |
| 41-T21-5-2.5% | 0.62063 | 0.49463 | 0.52057 | 0.48689 | 0.48433 | 0.52989 |
| 42-T21-6-2.5% | 0.49025 | 2.00E-05 | 0.47221 | 0.49507 | 0.37965 | 0.52569 |
| 43-T18-1-2.5% | 0.47667 | 0.43173 | 0.49475 | 0.49495 | 0.48245 | 0.49135 |
| 44-T18-2-2.5% | 0.46021 | 0.43753 | 0.49087 | 0.47217 | 0.43955 | 0.46207 |
| 45-T18-3-2.5% | 0.50703 | 0.44401 | 0.52321 | 0.52107 | 0.49731 | 0.51141 |
| 46-T13-1-2.5% | 0.49069 | 0.47959 | 0.49645 | 0.49907 | 0.50649 | 0.50489 |
| 47-T13-2-2.5% | 0.55195 | 0.60201 | 0.63029 | 0.64715 | 0.53017 | 0.53499 |
| 48-T13-3-2.5% | 0.50371 | 0.50401 | 0.50657 | 0.52835 | 0.46937 | 0.50233 |
| 49-T18M70%-T13M30% | 0.7868 | 0.65671 | 0.76166 | 0.65339 | 0.80938 | 0.63873 |
| 50-T18M80%-T13M20% | 0.86456 | 0.78006 | 0.8243 | 0.89088 | 0.76894 | 0.8107 |
| 51-T18M90%-T13M10% | 0.87678 | 0.63291 | 0.76284 | 0.68055 | 0.65465 | 0.63007 |
| 52-T13M70%-T21M30% | 0.83212 | 0.72523 | 0.63601 | 0.73627 | 0.65197 | 0.71233 |
| 53-T13M80%-T21M20% | 0.59415 | 0.55797 | 0.59911 | 0.50747 | 0.54045 | 0.63637 |
| 54-T13M90%-T21M10% | 0.64401 | 0.53687 | 0.65833 | 0.55881 | 0.53613 | 0.77358 |
| 55-T21M70%-T18M30% | 0.80454 | 0.74289 | 0.73503 | 0.76452 | 0.77804 | 0.77142 |
| 56-T21M80%-T18M20% | 0.7749 | 0.74359 | 0.7654 | 0.72865 | 0.75016 | 0.75104 |
| 57-T21M90%-T18M10% | 0.75174 | 0.74331 | 0.7542 | 0.74815 | 0.75574 | 0.73819 |
| 58-T2-10% | 0.49975 | 2.00E-05 | 0.19574 | 0.45429 | 0.30977 | 0.48797 |
| 59-T2-5% | 0.49431 | 2.00E-05 | 0.11206 | 0.45595 | 0.35495 | 0.46837 |
| 60-T3-10% | 0.48173 | 0.49771 | 2.00E-05 | 0.52211 | 0.51151 | 0.54889 |
| 61-T3-5% | 0.53475 | 0.50015 | 2.00E-05 | 0.51879 | 0.52005 | 0.50391 |
| 62-T4-10% | 0.69959 | 0.63217 | 0.65239 | 2.00E-05 | 0.66915 | 0.61581 |
| 63-T4-5% | 0.49531 | 0.49661 | 0.48695 | 2.00E-05 | 0.50247 | 0.52329 |
| 64-T5-10% | 0.67203 | 0.46709 | 0.63547 | 0.54993 | 2.00E-05 | 0.60939 |
| 65-T5-5% | 0.48521 | 0.52753 | 0.52981 | 0.46459 | 2.00E-05 | 0.47143 |
| 66-T6-10% | 0.66931 | 0.89642 | 0.67939 | 0.96142 | 0.93154 | 2.00E-05 |
| 67-T6-5% | 0.60379 | 0.74437 | 0.69357 | 0.81406 | 0.81162 | 2.00E-05 |
| 68-T7-10% | 0.58627 | 0.51855 | 0.58001 | 0.48791 | 0.51823 | 0.51747 |
| 69-T7-5% | 0.52709 | 0.50921 | 0.52825 | 0.50153 | 0.44903 | 0.62859 |
| 70-T8-10% | 0.58809 | 0.49087 | 0.50657 | 0.52095 | 0.49599 | 0.49443 |
| 71-T8-5% | 0.51425 | 0.50173 | 0.49049 | 0.48333 | 0.49823 | 0.47423 |
| 72-T9-10% | 0.62249 | 0.50447 | 0.49117 | 0.52343 | 0.60757 | 0.57239 |
| 73-T9-5% | 0.62455 | 0.49139 | 0.52807 | 0.53361 | 0.43639 | 0.48355 |
| 74-T10-10% | 0.51013 | 0.34123 | 0.41829 | 0.39457 | 0.32831 | 0.34899 |
| 75-T10-5% | 0.50205 | 0.47901 | 0.50201 | 0.50215 | 0.48147 | 0.42611 |
| 76-T11-10% | 0.90968 | 0.70307 | 0.71587 | 0.59717 | 0.63883 | 0.67389 |
| 77-T11-5% | 0.73441 | 0.53941 | 0.73641 | 0.7541 | 0.52147 | 0.58435 |
| 78-T12-10% | 0.57331 | 0.65185 | 0.72863 | 0.80024 | 0.85856 | 0.50879 |
| 79-T12-5% | 0.64487 | 0.84374 | 0.68141 | 0.85566 | 0.8919 | 0.79082 |
| 80-T14-10% | 0.71487 | 0.60935 | 0.75244 | 0.62705 | 0.66593 | 0.59685 |
| 81-T14-5% | 0.68695 | 0.59951 | 0.56743 | 0.65831 | 0.74411 | 0.50451 |
| 82-T15-10% | 0.77582 | 0.71917 | 0.71349 | 0.73149 | 0.73223 | 0.73681 |
| 83-T15-5% | 0.60303 | 0.53133 | 0.55403 | 0.62617 | 0.61481 | 0.45055 |
| 84-T16-10% | 0.68251 | 0.67401 | 0.58373 | 0.64081 | 0.56443 | 0.48673 |
| 85-T16-5% | 0.55655 | 0.57315 | 0.54209 | 0.54087 | 0.49315 | 0.57545 |
| 86-T17-10% | 0.76218 | 0.49939 | 0.65875 | 0.78068 | 0.69753 | 0.58249 |
| 87-T17-5% | 0.62745 | 0.51423 | 0.58827 | 0.57927 | 0.73797 | 0.56045 |
| 88-T19-10% | 0.9432 | 0.98088 | 0.86828 | 0.99052 | 0.88308 | 0.91326 |
| 89-T19-5% | 0.84834 | 0.86294 | 0.83282 | 0.93062 | 0.98754 | 0.82854 |
| 90-T20-10% | 0.76896 | 0.69359 | 0.74161 | 0.72751 | 0.66795 | 0.74251 |
| 91-T20-5% | 0.64817 | 0.64597 | 0.74613 | 0.72311 | 0.68167 | 0.68611 |
| 92-T22-10% | 0.72427 | 0.70783 | 0.64649 | 0.64781 | 0.74629 | 0.70585 |
| 93-T22-5% | 0.74077 | 0.72059 | 0.74711 | 0.75002 | 0.74413 | 0.74779 |

Table 6 below shows test probability values P of each of chromosome 7 to chromosome 12 corresponding to the 93 national reference materials according to embodiment two of the present invention.

| National reference | chr7_pv | chr8_pv | chr9_pv | chr10_pv | chr11_pv | chr12_pv |
|---|---|---|---|---|---|---|
| 1-T21-1-10% | 0.77446 | 0.78636 | 0.65981 | 0.65707 | 0.74085 | 0.74059 |
| 2-T21-2-10% | 0.81898 | 0.76084 | 0.72223 | 0.74989 | 0.73653 | 0.75054 |
| 3-T21-3-10% | 0.81672 | 0.7541 | 0.72451 | 0.75556 | 0.54047 | 0.64723 |
| 4-T21-4-10% | 0.81114 | 0.72583 | 0.75276 | 0.73399 | 0.41503 | 0.71589 |
| 5-T21-5-10% | 0.78668 | 0.75476 | 0.73263 | 0.73353 | 0.64061 | 0.73803 |
| 6-T21-6-10% | 0.88202 | 0.75602 | 0.75396 | 0.72633 | 0.74249 | 0.75172 |
| 7-T18-1-10% | 0.8263 | 0.69773 | 0.29323 | 0.48289 | 0.41709 | 0.48547 |
| 8-T18-2-10% | 0.61641 | 0.45965 | 0.34419 | 0.47797 | 0.44639 | 0.52279 |
| 9-T18-3-10% | 0.7601 | 0.76066 | 0.51431 | 0.49769 | 0.21836 | 0.58743 |
| 10-T13-1-10% | 0.69267 | 0.53133 | 0.48561 | 0.49873 | 0.49567 | 0.49925 |
| 11-T13-2-10% | 0.73889 | 0.72321 | 0.56657 | 0.60995 | 0.38931 | 0.64241 |
| 12-T13-3-10% | 0.85006 | 0.76756 | 0.39747 | 0.54707 | 0.42539 | 0.53273 |
| 13-T21-1-5% | 0.90036 | 0.63171 | 0.54853 | 0.46917 | 0.48109 | 0.49071 |
| 14-T21-2-5% | 0.8397 | 0.65145 | 0.41721 | 0.62261 | 0.37331 | 0.58477 |
| 15-T21-3-5% | 0.76394 | 0.58023 | 0.45307 | 0.58473 | 0.47967 | 0.64297 |
| 16-T21-4-5% | 0.78758 | 0.49601 | 0.49601 | 0.47911 | 0.63797 | 0.72431 |
| 17-T21-5-5% | 0.81824 | 0.82502 | 0.54425 | 0.47049 | 0.52229 | 0.63247 |
| 18-T21-6-5% | 0.81348 | 0.68219 | 0.44807 | 0.49157 | 0.35699 | 0.58123 |
| 19-T18-1-5% | 0.7923 | 0.65609 | 0.48201 | 0.60097 | 0.51693 | 0.58901 |
| 20-T18-2-5% | 0.8624 | 0.47731 | 0.41763 | 0.55041 | 0.41379 | 0.47989 |
| 21-T18-3-5% | 0.84664 | 0.63267 | 0.37285 | 0.59391 | 0.40031 | 0.49275 |
| 22-T13-1-5% | 0.78122 | 0.49207 | 0.45729 | 0.50131 | 0.33097 | 0.48503 |
| 23-T13-2-5% | 0.83924 | 0.69037 | 0.49689 | 0.47859 | 0.48509 | 0.49801 |
| 24-T13-3-5% | 0.72459 | 0.70571 | 0.42787 | 0.53269 | 0.40115 | 0.49757 |
| 25-T21-1-3.5% | 0.90976 | 0.70265 | 0.40981 | 0.46215 | 0.36377 | 0.55029 |
| 26-T21-2-3.5% | 0.91246 | 0.65927 | 0.59153 | 0.65757 | 0.51007 | 0.54765 |
| 27-T21-3-3.5% | 0.85554 | 0.59789 | 0.59407 | 0.46243 | 0.60501 | 0.51279 |
| 28-T21-4-3.5% | 0.93184 | 0.70947 | 0.66411 | 0.87382 | 0.48531 | 0.62765 |
| 29-T21-5-3.5% | 0.76934 | 0.49713 | 0.51101 | 0.52645 | 0.42031 | 0.59977 |
| 30-T21-6-3.5% | 0.91936 | 0.61343 | 0.54085 | 0.48715 | 0.50697 | 0.61715 |
| 31-T18-1-3.5% | 0.79612 | 0.61267 | 0.51397 | 0.52047 | 0.30105 | 0.53327 |
| 32-T18-2-3.5% | 0.78124 | 0.75878 | 0.57307 | 0.43937 | 0.48697 | 0.42833 |
| 33-T18-3-3.5% | 0.80604 | 0.43785 | 0.35327 | 0.54875 | 0.39979 | 0.31387 |
| 34-T13-1-3.5% | 0.91666 | 0.42021 | 0.14348 | 0.34963 | 0.30673 | 0.25919 |
| 35-T13-2-3.5% | 0.54789 | 0.52693 | 0.48957 | 0.25717 | 0.46911 | 0.46601 |
| 36-T13-3-3.5% | 0.86246 | 0.50761 | 0.26037 | 0.31203 | 0.46311 | 0.33201 |
| 37-T21-1-2.5% | 0.68537 | 0.49371 | 0.49191 | 0.48241 | 0.29491 | 0.51033 |
| 38-T21-2-2.5% | 0.70037 | 0.49157 | 0.47697 | 0.50155 | 0.47893 | 0.65933 |
| 39-T21-3-2.5% | 0.69559 | 0.49683 | 0.47031 | 0.47891 | 0.48567 | 0.51461 |
| 40-T21-4-2.5% | 0.7549 | 0.47823 | 0.53903 | 0.40481 | 0.47137 | 0.50149 |
| 41-T21-5-2.5% | 0.92422 | 0.48101 | 0.36351 | 0.47561 | 0.08674 | 0.43639 |
| 42-T21-6-2.5% | 0.80094 | 0.49323 | 0.26047 | 0.50381 | 0.19584 | 0.34985 |
| 43-T18-1-2.5% | 0.67809 | 0.48617 | 0.27301 | 0.49739 | 0.33805 | 0.43619 |
| 44-T18-2-2.5% | 0.51651 | 0.50423 | 0.44969 | 0.44313 | 0.20964 | 0.46497 |
| 45-T18-3-2.5% | 0.66559 | 0.48005 | 0.40651 | 0.52617 | 0.28317 | 0.37375 |
| 46-T13-1-2.5% | 0.58071 | 0.47937 | 0.44555 | 0.48557 | 0.17206 | 0.31477 |
| 47-T13-2-2.5% | 0.81384 | 0.45279 | 0.45741 | 0.49087 | 0.42605 | 0.48267 |
| 48-T13-3-2.5% | 0.82424 | 0.50897 | 0.44447 | 0.42005 | 0.47291 | 0.49889 |
| 49-T18M70%-T13M30% | 0.8665 | 0.63485 | 0.55437 | 0.66931 | 0.66533 | 0.58137 |
| 50-T18M80%-T13M20% | 0.92938 | 0.77406 | 0.74835 | 0.67251 | 0.64971 | 0.60519 |
| 51-T18M90%-T13M10% | 0.88748 | 0.64983 | 0.54453 | 0.42853 | 0.74229 | 0.57609 |
| 52-T13M70%-T21M30% | 0.87582 | 0.61111 | 0.48531 | 0.59115 | 0.55235 | 0.50193 |
| 53-T13M80%-T21M20% | 0.91692 | 0.58601 | 0.51571 | 0.63413 | 0.57315 | 0.56905 |
| 54-T13M90%-T21M10% | 0.80326 | 0.57209 | 0.46873 | 0.51137 | 0.45893 | 0.45357 |
| 55-T21M70%-T18M30% | 0.88374 | 0.74215 | 0.71351 | 0.62307 | 0.69045 | 0.74399 |
| 56-T21M80%-T18M20% | 0.82538 | 0.7545 | 0.74177 | 0.73769 | 0.72685 | 0.55943 |
| 57-T21M90%-T18M10% | 0.75416 | 0.75906 | 0.52149 | 0.69919 | 0.74773 | 0.71447 |
| 58-T2-10% | 0.47117 | 0.39569 | 0.36663 | 0.14608 | 0.20228 | 0.42773 |
| 59-T2-5% | 0.48449 | 0.41971 | 0.43601 | 0.17952 | 0.16086 | 0.44153 |
| 60-T3-10% | 0.57963 | 0.64119 | 0.39671 | 0.38989 | 0.42603 | 0.48209 |
| 61-T3-5% | 0.53611 | 0.50873 | 0.44797 | 0.47567 | 0.33579 | 0.51953 |
| 62-T4-10% | 0.9171 | 0.65097 | 0.52137 | 0.49493 | 0.57017 | 0.48037 |
| 63-T4-5% | 0.69331 | 0.51013 | 0.47477 | 0.37713 | 0.29311 | 0.48453 |
| 64-T5-10% | 0.86022 | 0.55543 | 0.45943 | 0.53991 | 0.70123 | 0.45881 |
| 65-T5-5% | 0.77592 | 0.52711 | 0.48981 | 0.60301 | 0.49133 | 0.60519 |
| 66-T6-10% | 0.9892 | 0.90336 | 0.57899 | 0.85412 | 0.32369 | 0.44057 |
| 67-T6-5% | 0.92226 | 0.77508 | 0.53733 | 0.68675 | 0.49661 | 0.59393 |
| 68-T7-10% | 2.00E-05 | 0.67649 | 0.42165 | 0.48721 | 0.47105 | 0.46911 |
| 69-T7-5% | 2.00E-05 | 0.51673 | 0.42521 | 0.46301 | 0.46485 | 0.49639 |
| 70-T8-10% | 0.74979 | 2.00E-05 | 0.44323 | 0.48663 | 0.55645 | 0.45693 |
| 71-T8-5% | 0.77766 | 2.00E-05 | 0.47237 | 0.46487 | 0.30873 | 0.54169 |
| 72-T9-10% | 0.83088 | 0.64815 | 2.00E-05 | 0.53787 | 0.45777 | 0.47009 |
| 73-T9-5% | 0.76462 | 0.56691 | 2.00E-05 | 0.53571 | 0.47725 | 0.46857 |
| 74-T10-10% | 0.44927 | 0.39653 | 0.34125 | 2.00E-05 | 0.33797 | 0.32979 |
| 75-T10-5% | 0.54093 | 0.53441 | 0.45323 | 2.00E-05 | 0.51395 | 0.30407 |
| 76-T11-10% | 0.92152 | 0.55269 | 0.50603 | 0.54931 | 2.00E-05 | 0.57575 |
| 77-T11-5% | 0.8804 | 0.73681 | 0.48419 | 0.49457 | 2.00E-05 | 0.51015 |
| 78-T12-10% | 0.74593 | 0.89302 | 0.34419 | 0.64271 | 0.73411 | 2.00E-05 |
| 79-T12-5% | 0.8703 | 0.8345 | 0.27965 | 0.55333 | 0.73389 | 2.00E-05 |
| 80-T14-10% | 0.8606 | 0.77234 | 0.45241 | 0.61143 | 0.60679 | 0.40847 |
| 81-T14-5% | 0.87492 | 0.53235 | 0.43239 | 0.58205 | 0.46537 | 0.46181 |
| 82-T15-10% | 0.83608 | 0.77128 | 0.44205 | 0.59581 | 0.60599 | 0.63673 |
| 83-T15-5% | 0.68313 | 0.55123 | 0.35559 | 0.45883 | 0.40725 | 0.39313 |
| 84-T16-10% | 0.87502 | 0.51347 | 0.49249 | 0.52175 | 0.41135 | 0.47253 |
| 85-T16-5% | 0.72439 | 0.58493 | 0.29165 | 0.47645 | 0.31459 | 0.57929 |
| 86-T17-10% | 0.77068 | 0.72977 | 0.45743 | 0.59017 | 0.52157 | 0.61727 |
| 87-T17-5% | 0.69567 | 0.43365 | 0.41817 | 0.49627 | 0.48099 | 0.48279 |
| 88-T19-10% | 0.99992 | 0.97396 | 0.62355 | 0.89214 | 0.71197 | 0.8855 |
| 89-T19-5% | 0.98808 | 0.94276 | 0.42079 | 0.79174 | 0.55123 | 0.81074 |
| 90-T20-10% | 0.80796 | 0.73567 | 0.73081 | 0.72107 | 0.74615 | 0.71265 |
| 91-T20-5% | 0.79152 | 0.68351 | 0.55897 | 0.72875 | 0.71131 | 0.55405 |
| 92-T22-10% | 0.77296 | 0.73623 | 0.52907 | 0.68643 | 0.65407 | 0.70727 |
| 93-T22-5% | 0.76546 | 0.65779 | 0.69425 | 0.69701 | 0.65885 | 0.50571 |

Table 7 below shows test probability values P of each of chromosome 13 to chromosome 17 corresponding to the 93 national reference materials according to embodiment two of the present invention.

| National reference | chr13_pv | chr14_pv | chr15_pv | chr16_pv | chr17_pv |
|---|---|---|---|---|---|
| 1-T21-1-10% | 0.75992 | 0.82666 | 0.74737 | 0.76584 | 0.77176 |
| 2-T21-2-10% | 0.7513 | 0.76904 | 0.8294 | 0.74037 | 0.74201 |
| 3-T21-3-10% | 0.76708 | 0.74919 | 0.77306 | 0.73877 | 0.78788 |
| 4-T21-4-10% | 0.78066 | 0.74103 | 0.75338 | 0.74175 | 0.74599 |
| 5-T21-5-10% | 0.74853 | 0.75104 | 0.76122 | 0.77116 | 0.74371 |
| 6-T21-6-10% | 0.84866 | 0.74043 | 0.79068 | 0.73963 | 0.78014 |
| 7-T18-1-10% | 0.84766 | 0.60321 | 0.78508 | 0.55737 | 0.53715 |
| 8-T18-2-10% | 0.49109 | 0.38447 | 0.66095 | 0.59981 | 0.85434 |
| 9-T18-3-10% | 0.62815 | 0.72725 | 0.69971 | 0.66413 | 0.78006 |
| 10-T13-1-10% | 2.00E-05 | 0.47391 | 0.79078 | 0.55507 | 0.46627 |
| 11-T13-2-10% | 2.00E-05 | 0.80262 | 0.64699 | 0.45905 | 0.46599 |
| 12-T13-3-10% | 2.00E-05 | 0.66963 | 0.57815 | 0.76932 | 0.47107 |
| 13-T21-1-5% | 0.7918 | 0.59065 | 0.73551 | 0.56967 | 0.56813 |
| 14-T21-2-5% | 0.91024 | 0.60031 | 0.74365 | 0.60689 | 0.50427 |
| 15-T21-3-5% | 0.78134 | 0.65037 | 0.79672 | 0.80282 | 0.80144 |
| 16-T21-4-5% | 0.73577 | 0.78376 | 0.82932 | 0.8379 | 0.56973 |
| 17-T21-5-5% | 0.75594 | 0.67745 | 0.79632 | 0.59847 | 0.45245 |
| 18-T21-6-5% | 0.90384 | 0.65713 | 0.68497 | 0.80554 | 0.57045 |
| 19-T18-1-5% | 0.83994 | 0.62205 | 0.66147 | 0.69167 | 0.36267 |
| 20-T18-2-5% | 0.78166 | 0.47995 | 0.55285 | 0.51957 | 0.61287 |
| 21-T18-3-5% | 0.69385 | 0.48955 | 0.52445 | 0.63551 | 0.29747 |
| 22-T13-1-5% | 2.00E-05 | 0.22204 | 0.59881 | 0.79762 | 0.31097 |
| 23-T13-2-5% | 2.00E-05 | 0.48895 | 0.63671 | 0.58607 | 0.51353 |
| 24-T13-3-5% | 2.00E-05 | 0.58969 | 0.52785 | 0.51861 | 0.46485 |
| 25-T21-1-3.5% | 0.8137 | 0.71695 | 0.72921 | 0.68059 | 0.61313 |
| 26-T21-2-3.5% | 0.72907 | 0.83584 | 0.69967 | 0.50053 | 0.48001 |
| 27-T21-3-3.5% | 0.84694 | 0.75058 | 0.79828 | 0.53289 | 0.46537 |
| 28-T21-4-3.5% | 0.96314 | 0.82488 | 0.79254 | 0.54635 | 0.37527 |
| 29-T21-5-3.5% | 0.80536 | 0.73053 | 0.59091 | 0.64997 | 0.51263 |
| 30-T21-6-3.5% | 0.74641 | 0.69833 | 0.67903 | 0.46409 | 0.46267 |
| 31-T18-1-3.5% | 0.95174 | 0.65821 | 0.55757 | 0.45389 | 0.45407 |
| 32-T18-2-3.5% | 0.88682 | 0.46573 | 0.54911 | 0.72111 | 0.44021 |
| 33-T18-3-3.5% | 0.60065 | 0.32405 | 0.46417 | 0.72445 | 0.39587 |
| 34-T13-1-3.5% | 0.00012 | 0.42581 | 0.64471 | 0.36879 | 0.25987 |
| 35-T13-2-3.5% | 2.00E-05 | 0.49647 | 0.48595 | 0.50301 | 0.45633 |
| 36-T13-3-3.5% | 2.00E-05 | 0.48423 | 0.63097 | 0.45233 | 0.44705 |
| 37-T21-1-2.5% | 0.70745 | 0.43095 | 0.61059 | 0.51571 | 0.47893 |
| 38-T21-2-2.5% | 0.74373 | 0.45907 | 0.46603 | 0.47853 | 0.49187 |
| 39-T21-3-2.5% | 0.47893 | 0.47475 | 0.49161 | 0.73057 | 0.49521 |
| 40-T21-4-2.5% | 0.56101 | 0.57969 | 0.50499 | 0.77572 | 0.62639 |
| 41-T21-5-2.5% | 0.78524 | 0.51219 | 0.55873 | 0.61805 | 0.50619 |
| 42-T21-6-2.5% | 0.49671 | 0.47363 | 0.52017 | 0.70255 | 0.50011 |
| 43-T18-1-2.5% | 0.51371 | 0.46945 | 0.46665 | 0.61915 | 0.45915 |
| 44-T18-2-2.5% | 0.54117 | 0.47153 | 0.49985 | 0.49887 | 0.49155 |
| 45-T18-3-2.5% | 0.62247 | 0.49455 | 0.60481 | 0.38327 | 0.47397 |
| 46-T13-1-2.5% | 0.00262 | 0.43153 | 0.48213 | 0.52717 | 0.16898 |
| 47-T13-2-2.5% | 0.01158 | 0.48201 | 0.47073 | 0.43803 | 0.45835 |
| 48-T13-3-2.5% | 0.0002 | 0.54977 | 0.50709 | 0.50619 | 0.49789 |
| 49-T18M70%-T13M30% | 0.00016 | 0.62045 | 0.62705 | 0.56751 | 0.74451 |
| 50-T18M80%-T13M20% | 0.04554 | 0.83208 | 0.91724 | 0.61881 | 0.53919 |
| 51-T18M90%-T13M10% | 0.42637 | 0.48657 | 0.86078 | 0.67597 | 0.43487 |
| 52-T13M70%-T21M30% | 2.00E-05 | 0.70037 | 0.8674 | 0.70861 | 0.49973 |
| 53-T13M80%-T21M20% | 2.00E-05 | 0.71693 | 0.7972 | 0.7853 | 0.50927 |
| 54-T13M90%-T21M10% | 2.00E-05 | 0.63991 | 0.54789 | 0.72453 | 0.73397 |
| 55-T21M70%-T18M30% | 0.84984 | 0.78936 | 0.80816 | 0.76902 | 0.54721 |
| 56-T21M80%-T18M20% | 0.76166 | 0.76042 | 0.85858 | 0.70555 | 0.74637 |
| 57-T21M90%-T18M10% | 0.7665 | 0.74869 | 0.80522 | 0.74003 | 0.73937 |
| 58-T2-10% | 0.49469 | 0.56235 | 0.59619 | 0.53093 | 0.55787 |
| 59-T2-5% | 0.49687 | 0.50949 | 0.49795 | 0.60647 | 0.63431 |
| 60-T3-10% | 0.62527 | 0.47025 | 0.82708 | 0.59847 | 0.51855 |
| 61-T3-5% | 0.50223 | 0.49329 | 0.52065 | 0.48509 | 0.31673 |
| 62-T4-10% | 0.76734 | 0.54037 | 0.74861 | 0.8118 | 0.56161 |
| 63-T4-5% | 0.73845 | 0.53859 | 0.50485 | 0.54135 | 0.46389 |
| 64-T5-10% | 0.57415 | 0.51161 | 0.78952 | 0.66351 | 0.40681 |
| 65-T5-5% | 0.65273 | 0.58181 | 0.55831 | 0.52687 | 0.45875 |
| 66-T6-10% | 0.9814 | 0.78422 | 0.95344 | 0.56221 | 0.30915 |
| 67-T6-5% | 0.85884 | 0.51729 | 0.8993 | 0.49393 | 0.20596 |
| 68-T7-10% | 0.54419 | 0.52267 | 0.54747 | 0.72283 | 0.42891 |
| 69-T7-5% | 0.75584 | 0.49231 | 0.53659 | 0.52315 | 0.1907 |
| 70-T8-10% | 0.76156 | 0.43901 | 0.68693 | 0.63483 | 0.57475 |
| 71-T8-5% | 0.50253 | 0.48021 | 0.73555 | 0.47807 | 0.49853 |
| 72-T9-10% | 0.67397 | 0.69035 | 0.74489 | 0.64877 | 0.62477 |
| 73-T9-5% | 0.61691 | 0.65749 | 0.65067 | 0.49071 | 0.46179 |
| 74-T10-10% | 0.53163 | 0.29089 | 0.71903 | 0.48021 | 0.70493 |
| 75-T10-5% | 0.51083 | 0.47463 | 0.49469 | 0.66569 | 0.48539 |
| 76-T11-10% | 0.86112 | 0.45853 | 0.78658 | 0.44267 | 0.52559 |
| 77-T11-5% | 0.85318 | 0.62783 | 0.61049 | 0.48613 | 0.32869 |
| 78-T12-10% | 0.9119 | 0.61803 | 0.61047 | 0.913 | 0.20518 |
| 79-T12-5% | 0.83108 | 0.74025 | 0.80798 | 0.79842 | 0.31651 |
| 80-T14-10% | 0.8295 | 2.00E-05 | 0.67295 | 0.64811 | 0.70193 |
| 81-T14-5% | 0.68897 | 2.00E-05 | 0.7679 | 0.64507 | 0.48099 |
| 82-T15-10% | 0.77856 | 0.64817 | 2.00E-05 | 0.60771 | 0.45981 |
| 83-T15-5% | 0.72311 | 0.66647 | 2.00E-05 | 0.52759 | 0.48229 |
| 84-T16-10% | 0.7993 | 0.7931 | 0.62325 | 2.00E-05 | 0.64695 |
| 85-T16-5% | 0.8614 | 0.50525 | 0.64935 | 2.00E-05 | 0.47327 |
| 86-T17-10% | 0.84092 | 0.81338 | 0.66111 | 0.62557 | 2.00E-05 |
| 87-T17-5% | 0.68601 | 0.44339 | 0.60251 | 0.82068 | 2.00E-05 |
| 88-T19-10% | 0.9998 | 0.93146 | 0.98562 | 0.99262 | 0.71127 |
| 89-T19-5% | 0.999 | 0.82168 | 0.97564 | 0.92754 | 0.87382 |
| 90-T20-10% | 0.84878 | 0.73851 | 0.80684 | 0.69447 | 0.42469 |
| 91-T20-5% | 0.75808 | 0.74641 | 0.60321 | 0.51149 | 0.59007 |
| 92-T22-10% | 0.76846 | 0.71853 | 0.74773 | 0.74183 | 0.75224 |
| 93-T22-5% | 0.82604 | 0.74073 | 0.71773 | 0.56923 | 0.54631 |

Table 8 below shows test probability values P of each of chromosome 18 to chromosome 22 corresponding to the 93 national reference materials according to embodiment two of the present invention.

| National reference | chr18_pv | chr19_pv | chr20_pv | chr21_pv | chr22_pv |
|---|---|---|---|---|---|
| 1-T21-1-10% | 0.56847 | 0.99992 | 0.78232 | 2.00E-05 | 0.87806 |
| 2-T21-2-10% | 0.74585 | 0.99922 | 0.78038 | 2.00E-05 | 0.919 |
| 3-T21-3-10% | 0.75162 | 1 | 0.82774 | 2.00E-05 | 0.8496 |
| 4-T21-4-10% | 0.72667 | 1 | 0.75068 | 2.00E-05 | 0.93746 |
| 5-T21-5-10% | 0.74923 | 1 | 0.75668 | 2.00E-05 | 0.8007 |
| 6-T21-6-10% | 0.8376 | 0.99904 | 0.83308 | 2.00E-05 | 0.94594 |
| 7-T18-1-10% | 2.00E-05 | 0.99922 | 0.74467 | 0.78996 | 0.9667 |
| 8-T18-2-10% | 2.00E-05 | 1 | 0.64947 | 0.50451 | 0.74487 |
| 9-T18-3-10% | 2.00E-05 | 1 | 0.68997 | 0.52885 | 0.78418 |
| 10-T13-1-10% | 0.51261 | 1 | 0.73691 | 0.66575 | 0.49713 |
| 11-T13-2-10% | 0.71073 | 0.99182 | 0.73441 | 0.84736 | 0.7906 |
| 12-T13-3-10% | 0.47595 | 1 | 0.64525 | 0.89928 | 0.71055 |
| 13-T21-1-5% | 0.69207 | 1 | 0.59835 | 2.00E-05 | 0.8105 |
| 14-T21-2-5% | 0.64635 | 1 | 0.7819 | 2.00E-05 | 0.74571 |
| 15-T21-3-5% | 0.61063 | 1 | 0.83808 | 2.00E-05 | 0.74389 |
| 16-T21-4-5% | 0.61077 | 1 | 0.74609 | 2.00E-05 | 0.84762 |
| 17-T21-5-5% | 0.76798 | 0.99998 | 0.66719 | 2.00E-05 | 0.67057 |
| 18-T21-6-5% | 0.54371 | 1 | 0.75354 | 2.00E-05 | 0.8463 |
| 19-T18-1-5% | 2.00E-05 | 0.9989 | 0.43615 | 0.89324 | 0.52067 |
| 20-T18-2-5% | 2.00E-05 | 0.99996 | 0.42891 | 0.69411 | 0.8397 |
| 21-T18-3-5% | 2.00E-05 | 1 | 0.55337 | 0.86078 | 0.62325 |
| 22-T13-1-5% | 0.50675 | 1 | 0.64027 | 0.65341 | 0.85218 |
| 23-T13-2-5% | 0.70469 | 1 | 0.62265 | 0.48989 | 0.79188 |
| 24-T13-3-5% | 0.61619 | 0.9992 | 0.66859 | 0.9273 | 0.54261 |
| 25-T21-1-3.5% | 0.58417 | 1 | 0.68025 | 2.00E-05 | 0.84822 |
| 26-T21-2-3.5% | 0.61419 | 0.99998 | 0.64805 | 2.00E-05 | 0.72575 |
| 27-T21-3-3.5% | 0.56831 | 0.99998 | 0.68583 | 2.00E-05 | 0.79782 |
| 28-T21-4-3.5% | 0.80398 | 0.99894 | 0.52085 | 2.00E-05 | 0.40955 |
| 29-T21-5-3.5% | 0.60189 | 1 | 0.54907 | 2.00E-05 | 0.64501 |
| 30-T21-6-3.5% | 0.62295 | 1 | 0.47273 | 2.00E-05 | 0.76418 |
| 31-T18-1-3.5% | 2.00E-05 | 0.9995 | 0.44937 | 0.82454 | 0.42107 |
| 32-T18-2-3.5% | 2.00E-05 | 1 | 0.84574 | 0.46359 | 0.67669 |
| 33-T18-3-3.5% | 2.00E-05 | 1 | 0.50449 | 0.95452 | 0.57459 |
| 34-T13-1-3.5% | 0.40057 | 1 | 0.49527 | 0.92448 | 0.98152 |
| 35-T13-2-3.5% | 0.55811 | 1 | 0.49983 | 0.58165 | 0.72699 |
| 36-T13-3-3.5% | 0.53873 | 1 | 0.58507 | 0.89476 | 0.51167 |
| 37-T21-1-2.5% | 0.48797 | 1 | 0.62783 | 2.00E-05 | 0.89472 |
| 38-T21-2-2.5% | 0.53501 | 1 | 0.90978 | 0.00028 | 0.59843 |
| 39-T21-3-2.5% | 0.56935 | 1 | 0.66251 | 6.00E-05 | 0.75766 |
| 40-T21-4-2.5% | 0.48891 | 1 | 0.60649 | 2.00E-05 | 0.61859 |
| 41-T21-5-2.5% | 0.47215 | 1 | 0.69267 | 0.03206 | 0.51009 |
| 42-T21-6-2.5% | 0.61005 | 1 | 0.48991 | 0.0002 | 0.73039 |
| 43-T18-1-2.5% | 2.00E-05 | 1 | 0.55575 | 0.52397 | 0.79346 |
| 44-T18-2-2.5% | 2.00E-05 | 1 | 0.53341 | 0.68803 | 0.8664 |
| 45-T18-3-2.5% | 2.00E-05 | 1 | 0.46863 | 0.8545 | 0.47719 |
| 46-T13-1-2.5% | 0.48251 | 1 | 0.53219 | 0.68453 | 0.87258 |
| 47-T13-2-2.5% | 0.52971 | 1 | 0.48065 | 0.80014 | 0.51111 |
| 48-T13-3-2.5% | 0.49391 | 1 | 0.61601 | 0.50007 | 0.50353 |
| 49-T18M70%-T13M30% | 2.00E-05 | 0.98638 | 0.74151 | 0.92372 | 0.82224 |
| 50-T18M80%-T13M20% | 2.00E-05 | 0.86874 | 0.80264 | 0.93904 | 0.49167 |
| 51-T18M90%-T13M10% | 2.00E-05 | 0.997 | 0.83184 | 0.73163 | 0.93384 |
| 52-T13M70%-T21M30% | 0.58307 | 1 | 0.69587 | 0.00026 | 0.73183 |
| 53-T13M80%-T21M20% | 0.50477 | 1 | 0.51439 | 0.06352 | 0.83512 |
| 54-T13M90%-T21M10% | 0.54719 | 1 | 0.51321 | 0.47485 | 0.97674 |
| 55-T21M70%-T18M30% | 0.00266 | 0.99966 | 0.7794 | 2.00E-05 | 0.96892 |
| 56-T21M80%-T18M20% | 0.17966 | 1 | 0.74781 | 2.00E-05 | 0.94434 |
| 57-T21M90%-T18M10% | 0.27589 | 1 | 0.8044 | 2.00E-05 | 0.9299 |
| 58-T2-10% | 0.52163 | 1 | 0.57631 | 0.66051 | 0.73531 |
| 59-T2-5% | 0.49103 | 1 | 0.50457 | 0.51257 | 0.74123 |
| 60-T3-10% | 0.68895 | 0.99994 | 0.62671 | 0.68153 | 0.72183 |
| 61-T3-5% | 0.55041 | 1 | 0.69997 | 0.63953 | 0.74073 |
| 62-T4-10% | 0.78962 | 0.99982 | 0.61395 | 0.48301 | 0.48511 |
| 63-T4-5% | 0.46165 | 0.99998 | 0.52897 | 0.60015 | 0.87794 |
| 64-T5-10% | 0.46481 | 0.96992 | 0.75262 | 0.72835 | 0.86112 |
| 65-T5-5% | 0.46853 | 0.99988 | 0.60973 | 0.73883 | 0.55791 |
| 66-T6-10% | 0.78 | 6.00E-05 | 0.88376 | 0.9375 | 0.66127 |
| 67-T6-5% | 0.68001 | 0.15696 | 0.58731 | 0.86104 | 0.55143 |
| 68-T7-10% | 0.70217 | 1 | 0.63551 | 0.73865 | 0.55819 |
| 69-T7-5% | 0.42851 | 1 | 0.61481 | 0.8128 | 0.69285 |
| 70-T8-10% | 0.46595 | 0.99998 | 0.65695 | 0.66321 | 0.83262 |
| 71-T8-5% | 0.50959 | 1 | 0.71531 | 0.68553 | 0.77652 |
| 72-T9-10% | 0.51967 | 1 | 0.83036 | 0.68365 | 0.62511 |
| 73-T9-5% | 0.55181 | 0.99996 | 0.56637 | 0.69143 | 0.73933 |
| 74-T10-10% | 0.40969 | 0.99998 | 0.52147 | 0.9784 | 0.94056 |
| 75-T10-5% | 0.47787 | 1 | 0.53185 | 0.47371 | 0.98682 |
| 76-T11-10% | 0.47573 | 0.99922 | 0.51481 | 0.68251 | 0.862 |
| 77-T11-5% | 0.60297 | 0.98876 | 0.52847 | 0.56919 | 0.88664 |
| 78-T12-10% | 0.87524 | 0.15882 | 0.96422 | 0.91688 | 0.7977 |
| 79-T12-5% | 0.90396 | 0.29647 | 0.78056 | 0.86726 | 0.18332 |
| 80-T14-10% | 0.55995 | 0.99996 | 0.7696 | 0.72973 | 0.78744 |
| 81-T14-5% | 0.58953 | 0.99996 | 0.59731 | 0.55881 | 0.58189 |
| 82-T15-10% | 0.71427 | 0.92568 | 0.87414 | 0.86998 | 0.77732 |
| 83-T15-5% | 0.31615 | 0.99992 | 0.7887 | 0.83308 | 0.89504 |
| 84-T16-10% | 0.74713 | 0.99836 | 0.72409 | 0.7804 | 0.94618 |
| 85-T16-5% | 0.46297 | 0.99976 | 0.57723 | 0.74235 | 0.95606 |
| 86-T17-10% | 0.71837 | 0.77556 | 0.89544 | 0.93898 | 0.87474 |
| 87-T17-5% | 0.54053 | 0.99984 | 0.72527 | 0.77456 | 0.8397 |
| 88-T19-10% | 0.99998 | 2.00E-05 | 0.99992 | 0.99996 | 1 |
| 89-T19-5% | 0.94138 | 2.00E-05 | 0.85888 | 0.99974 | 0.99996 |
| 90-T20-10% | 0.70669 | 0.88546 | 2.00E-05 | 0.76932 | 0.8412 |
| 91-T20-5% | 0.71477 | 0.96996 | 2.00E-05 | 0.82934 | 0.73055 |
| 92-T22-10% | 0.772 | 0.99974 | 0.80792 | 0.74869 | 2.00E-05 |
| 93-T22-5% | 0.7805 | 0.99988 | 0.75242 | 0.74871 | 2.00E-05 |

In the above Table 5 to Table 8, the leftmost column represents the sample types of the national reference materials, and the other columns represent the test probability values P of different human chromosomes corresponding to the 93 national reference materials. For example, in "chr1_pv", "chr1" represents chromosome 1 and "pv" represents the test probability value P, and in "41-T21-5-2.5%", "41" represents a number of a national reference material, "T21" represents a positive chromosome in the sample, that is, an aneuploid chromosome, "5" represents that "T21", as the positive chromosome, appears in at least five national reference materials, and "2.5%" represents the preset concentration of "T21".

Detection statistics corresponding to the above Table 5 to Table 8 are shown in Table 9 below.

| | Positive | Negative | Total |
|---|---|---|---|
| Detected to be positive | 83 | 3 | 86 |
| Detected to be negative | 10 | 30 | 40 |
| Total | 93 | 33 | 126 |

"83" denotes the number of samples detected to be positive among true positive samples, "10" denotes the number of samples detected to be negative among the true positive samples, "93" denotes the number of the true positive samples, "3" denotes the number of samples detected to be positive among true negative samples, "30" denotes the number of samples detected to be negative among the true negative samples, "33" denotes the number of the true negative samples, "86" denotes the number of samples detected to be positive, "40" denotes the number of samples detected to be negative, and "126" denotes the total number of samples.

The detection performance of the above Table 5 to Table 8 is shown in Table 10 below.

| Positive Predictive Value | Negative Predictive Value | Sensitivity | Specificity | Youden's Index |
|---|---|---|---|---|
| 96.51% | 75.00% | 89.25% | 90.91% | 80.16% |

The positive predictive value refers to a proportion of true positive samples to the samples detected to be positive, the negative predictive value refers to a proportion of true negative samples to the samples detected to be negative, the sensitivity refers to a proportion of the samples detected to be positive among the true positive samples, the specificity refers to a proportion of the samples detected to be negative among the true negative samples, and Youden's index = sensitivity + specificity - 1. The more Youden's index approaches 1, the better the detection performance.

After verification, the method for detecting chromosomal aneuploidy according to the embodiments of the present invention can detect a national reference material with a preset concentration greater than or equal to 5% and the detection performance meets the detection performance requirement of the national reference materials.

It is to be noted that the collection, use, storage, sharing, transfer and other processing of personal information of a user, which are involved in the technical solutions of the present invention, are all in compliance with relevant laws and regulations, and the notification to the user and the agreement or authorization of the user are required; and where applicable, the personal information of the user is subjected to technical processing including de-identification and/or anonymization and/or encryption.

The following are embodiments of an apparatus for detecting chromosomal aneuploidy according to an embodiment of the present invention. The apparatus and the method for detecting chromosomal aneuploidy in the preceding embodiments belong to the same inventive concept. For details not described in the embodiments of the apparatus for detecting chromosomal aneuploidy, reference may be made to the content about the method for detecting chromosomal aneuploidy in the preceding embodiments.

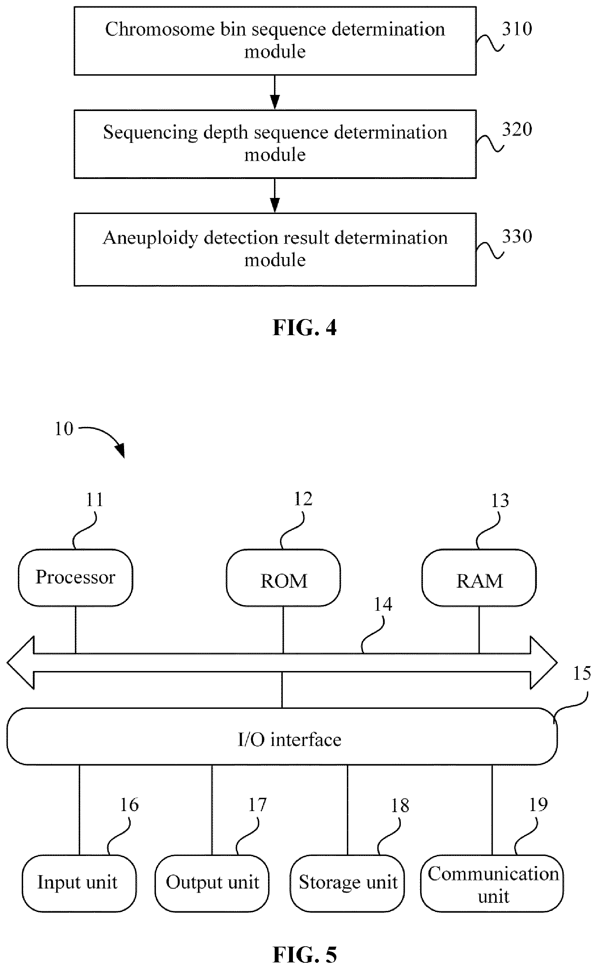

FIG. 4 is a structure diagram of an apparatus for detecting chromosomal aneuploidy according to an embodiment of the present invention. As shown in FIG. 4, the apparatus includes a chromosome bin sequence determination module 310, a sequencing depth sequence determination module 320 and an aneuploidy detection result determination module 330.

The chromosome bin sequence determination module 310 is configured to determine a chromosome bin sequence of a chromosome under test according to reference genome nucleic acid data of a human reference genome, where the chromosome bin sequence includes at least one bin number ratio, and each bin number ratio is the ratio of the number of nucleic acid bins of the chromosome under test in the human reference genome to the number of nucleic acid bins of a respective one of at least one preset chromosome in the human reference genome.

The sequencing depth sequence determination module 320 is configured to determine a sequencing depth sequence of the chromosome under test according to whole genome sequencing data of a nucleic acid sample under test, where the sequencing depth sequence includes at least one sequencing depth parameter, and each sequencing depth parameter represents a functional relationship between a sequencing depth of the chromosome under test in the nucleic acid sample under test and a sequencing depth of a respective one of the at least one preset chromosome in the nucleic acid sample under test.

The aneuploidy detection result determination module 330 is configured to, according to the chromosome bin sequence and the sequencing depth sequence, perform a non-parametric test to obtain an aneuploidy detection result of the chromosome under test in the nucleic acid sample under test.

For example, a source of the human reference genome may include NCBI database version GRCh36, GRCh37 or GRCh38, UCSC database version hg18, hg19 or hg38. The source of the human reference genome is not limited herein and may be customized according to actual requirements.

In the embodiments of the present application, nucleic acid data are used for representing nucleic acid sequences and may be standard sequences of the human reference genome (for example, the reference genome nucleic acid data) or sequences of a nucleic acid sample obtained through sequencing (for example, whole genome sequencing data). For example, the reference genome nucleic acid data in the embodiments of the present application refer to the standard sequences of the human reference genome, that is, sequences corresponding to real sequences of the human reference genome. For example, the reference genome nucleic acid data include at least a chromosome nucleic acid datum of the chromosome under test and a chromosome nucleic acid datum of each of the at least one preset chromosome. The chromosome under test is used for representing a human chromosome detected for an aneuploidy, and each preset chromosome is used for representing another human chromosome excluding the chromosome under test. In the embodiments of the present application, each chromosome under test corresponds to a group of preset chromosomes, and characteristic data of the chromosome under test are acquired based on the group of preset chromosomes, such as the number of bins and the sequencing depth. For each chromosome under test, the selection of the preset chromosomes is not strictly limited and may be set according to a target requirement to be met in the detection and based on the method according to the embodiments of the present application. For example, the chromosome under test may be chromosome 21, and the preset chromosomes include chromosome 1, chromosome 2 and chromosome 3.

In an exemplary embodiment, the chromosome bin sequence represents a proportional function model of nucleic acid bins of the chromosome under test and the group of preset chromosomes in the human reference genome. In this embodiment, the chromosome bin sequence includes the at least one bin number ratio, and each bin number ratio is the ratio of the number of nucleic acid bins of the chromosome under test in the human reference genome to the number of nucleic acid bins of one respective preset chromosome in the human reference genome.

The number of nucleic acid bins may be used for representing the number of nucleic acid bins included in the chromosome nucleic acid datum of the chromosome under test or the preset chromosome in the human reference genome. Bin division is performed on the chromosome nucleic acid datum according to a bin division rule so that the nucleic acid bins are obtained, and a bin position of each nucleic acid bin in the chromosome nucleic acid datum is unique.

In an optional embodiment, the chromosome bin sequence determination module 310 includes a reference chromosome nucleic acid datum acquisition unit, a nucleic acid bin number determination unit and a chromosome bin sequence determination unit.

The reference chromosome nucleic acid datum acquisition unit is configured to acquire, from the reference genome nucleic acid data, a reference chromosome nucleic acid datum of the chromosome under test and a reference chromosome nucleic acid datum of each of the at least one preset chromosome.

The nucleic acid bin number determination unit is configured to, for each reference chromosome nucleic acid datum, perform the bin division on the reference chromosome nucleic acid datum according to the bin division rule and determine, according to a bin division result, the number of nucleic acid bins of the chromosome under test and the number of nucleic acid bins of each preset chromosome.

The chromosome bin sequence determination unit is configured to determine the chromosome bin sequence of the chromosome under test according to the number of nucleic acid bins of the chromosome under test and the number of nucleic acid bins of each preset chromosome.

In an exemplary embodiment, the reference chromosome nucleic acid datum is a nucleic acid sequence datum corresponding to the chromosome under test or a nucleic acid sequence datum corresponding to the preset chromosome in the human reference genome. For example, assuming that the chromosome under test is chromosome 18, the reference chromosome nucleic acid datum is a nucleic acid sequence datum corresponding to chromosome 18 in the reference genome nucleic acid data of the human reference genome.

In an optional embodiment, the bin division rule includes a preset bin length and an interval between bins, where the preset bin length is used for representing a bin sequence length of a nucleic acid bin obtained through division. A specific parameter value of the preset bin length is not limited herein and may be customized according to the actual requirements. For example, the preset bin length is, but is not limited to, 20 kbp.

In an exemplary embodiment, the interval between bins is used for representing the length of a nucleic acid sequence between two adjacent nucleic acid bins. For example, the interval between bins may be -1 kb, 0 kb or 1 kb, where "-1 kb" indicates that two adjacent nucleic acid bins have an overlap of a nucleic acid sequence of 1 kb, "0 kb" indicates that no nucleic acid sequence exists as an overlap or interval between two adjacent nucleic acid bins, and "1 kb" indicates that a nucleic acid sequence of 1 kb exists as an interval between two adjacent nucleic acid bins. A specific parameter value of the interval between bins is not limited herein and may be customized according to the actual requirements.

In an optional embodiment, the nucleic acid bin number determination unit is configured to perform a deletion operation on a nucleic acid bin not including any known bases in the bin division result; and count remaining nucleic acid bins in the bin division result after the deletion operation to obtain the number of nucleic acid bins of the chromosome under test and the number of nucleic acid bins of each preset chromosome.

In an exemplary embodiment, nucleic acid bins in the bin division result are traversed. If the nucleic acid bin does not include any known bases, it indicates that the nucleic acid bin includes all unknown bases, and the nucleic acid bin is deleted from the bin division result.

Such setting has the following advantage: the nucleic acid bin including all the unknown bases is prevented from causing noise interference to the accuracy of the number of nucleic acid bins counted subsequently and the sequencing depth, further ensuring the accuracy of the aneuploidy detection result.

For example, the bin number ratio of the chromosome under test i and the preset chromosome *j* may be represented as rᵢⱼ = Lᵢ/Lⱼ, where *i* ≠ *j,* Lᵢ denotes the number of nucleic acid bins of the chromosome under test i, and Lⱼ denotes the number of nucleic acid bins of the preset chromosome *j*. For example, a chromosome bin sequence R₁ of chromosome 1 may be represented as R₁ = [r₁₂, r₁₃, r₁₄, ..., r₁ⱼ].

For example, a type of the nucleic acid sample under test is not strictly limited and may be any one including complete human DNA, where complete DNA refers to DNA that is not damaged in a sampling process and after sampling. For example, the nucleic acid sample under test may be a blood sample, a urine sample, a cell sample, a mucus sample or a tissue sample. A source of the nucleic acid sample under test has no effect on the method for detecting chromosomal aneuploidy and a detection result of chromosomal aneuploidy. Therefore, the source of the nucleic acid sample under test is not limited in the embodiments of the present application and may be customized according to the actual requirements.

In the embodiments of the present application, the whole genome sequencing data of the nucleic acid sample under test are nucleic acid sequence data obtained after whole genome sequencing is performed on the nucleic acid sample under test. Specifically, the whole genome sequencing data include a chromosome sequencing datum of the chromosome under test and a chromosome sequencing datum of each of the at least one preset chromosome. The chromosome sequencing datum represents all nucleic acid data included in a chromosome in the unit of chromosome.

In an optional embodiment, the whole genome sequencing data of the nucleic acid sample under test may be obtained through a whole genome sequencing data determination module. The whole genome sequencing data determination module is configured to extract a free nucleic acid from the nucleic acid sample under test; perform PCR amplification on the free nucleic acid and perform sample pretreatment to obtain a nucleic acid library; and perform the whole genome sequencing on the nucleic acid library to obtain the whole genome sequencing data of the nucleic acid sample under test.

For example, the PCR amplification is performed on the free nucleic acid by using a PCR nucleic acid amplifier, and the nucleic acid library is built according to the amplified free nucleic acid by using a chromosomal aneuploidy detection kit. A sequencing technology used for the whole genome sequencing includes, but is not limited to, a second-generation sequencing technology, a nanopore sequencing technology or a third-generation sequencing technology. The sequencing technology used for the whole genome sequencing is not limited herein and may be customized according to the actual requirements.

In an exemplary embodiment, the sequencing depth sequence represents a function model of sequencing depths of the chromosome under test and the group of preset chromosomes in the nucleic acid sample under test. In this embodiment, the sequencing depth sequence includes the at least one sequencing depth parameter, and each sequencing depth parameter represents the functional relationship between the sequencing depth of the chromosome under test in the nucleic acid sample under test and the sequencing depth of one respective preset chromosome in the nucleic acid sample under test. The meaning of the sequencing depth is as described above and is not repeated here.

In an optional embodiment, the sequencing depth sequence determination module 320 includes a chromosome sequencing datum acquisition unit, a sequencing depth determination unit and a sequencing depth sequence determination unit.

The chromosome sequencing datum acquisition unit is configured to acquire, from the whole genome sequencing data, the chromosome sequencing datum of the chromosome under test and the chromosome sequencing datum of each of the at least one preset chromosome.

The sequencing depth determination unit is configured to, for each chromosome sequencing datum, perform sequence alignment on the chromosome sequencing datum and at least one nucleic acid bin of a respective chromosome, determine the number of nucleic acid sequences in an alignment datum of each nucleic acid bin, and use the number of nucleic acid sequences in alignment data of the at least one nucleic acid bin as a sequencing depth of the respective chromosome.

The sequencing depth sequence determination unit is configured to determine the sequencing depth sequence of the chromosome under test according to the sequencing depth of the chromosome under test and a sequencing depth of each preset chromosome.

In an exemplary embodiment, the chromosome sequencing datum is a nucleic acid sequence datum corresponding to the chromosome under test or a nucleic acid sequence datum corresponding to the preset chromosome in the nucleic acid sample under test.

For example, assuming that the chromosome under test is chromosome 18, the chromosome sequencing datum is a nucleic acid sequence datum corresponding to chromosome 18 in the whole genome sequencing data of the nucleic acid sample under test, and the sequence alignment is performed on the chromosome sequencing datum of chromosome 18 and a nucleic acid bin of chromosome 18, where the nucleic acid bin of chromosome 18 is a nucleic acid bin counted to obtain the number of nucleic acid bins in S110.

For example, an alignment tool used in the alignment operation includes, but is not limited to, a TMAP tool, a BWA tool, an SOAP tool or SAMtools. The alignment tool used in the alignment operation is not limited herein and may be customized according to the actual requirements.

Specifically, the number of nucleic acid sequences is the number of nucleic acid fragments in each chromosome sequencing datum and aligned to a specified nucleic acid bin and may represent the distribution of the nucleic acid fragments in the specified nucleic acid bin.

In an optional embodiment, the sequencing depth determination unit is configured to acquire an initial number of sequences in the alignment datum of each nucleic acid bin and perform a correction operation on the initial number of sequences to obtain the number of nucleic acid sequences in the alignment datum of each nucleic acid bin.

In an exemplary embodiment, the initial number of sequences is the initial number of nucleic acid fragments in the chromosome sequencing datum and aligned to a specified nucleic acid bin, and the number of nucleic acid sequences is the corrected number of nucleic acid fragments in the chromosome sequencing datum and aligned to the specified nucleic acid bin.

In an optional embodiment, the correction operation includes at least one of effective base length correction, outlier correction, mappability correction or GC-content correction. A mappability value may be used for representing an alignment ability of the alignment tool to correctly align the chromosome sequencing datum to a nucleic acid bin in the human reference genome. The mappability correction refers to local polynomial regression fitting correction performed on the initial number of sequences in the alignment datum of the nucleic acid bin according to the mappability value. Since the initial number of sequences acquired from the alignment datum of the nucleic acid bin with a high GC content or a low GC content is less than the initial number of sequences acquired from the alignment datum of the nucleic acid bin with an intermediate GC content, the GC-content correction refers to normalization correction or local polynomial regression fitting correction performed on the initial number of sequences in the alignment datum of the nucleic acid bin according to the GC content of the alignment datum of the nucleic acid bin.

Such setting has the following advantage: different effective base lengths, different outliers, different mappability values and different GC contents are prevented from causing error interference to the sequencing depth of the chromosome, further improving the accuracy of the aneuploidy detection result.

In an optional embodiment, the sequencing depth sequence determination unit includes a reference sequencing depth ratio determination subunit and a sequencing depth sequence determination subunit.

The reference sequencing depth ratio determination subunit is configured to determine at least one reference sequencing depth ratio according to the sequencing depth of the chromosome under test and the sequencing depth of each preset chromosome, where each reference sequencing depth ratio is the ratio of the sequencing depth of the chromosome under test to a sequencing depth of one respective preset chromosome.

The sequencing depth sequence determination subunit is configured to determine the sequencing depth sequence of the chromosome under test according to the at least one reference sequencing depth ratio.

In an optional embodiment, the sequencing depth parameter is the reference sequencing depth ratio. For example, the reference sequencing depth ratio of the chromosome under test i and the preset chromosome *j* may be represented as tᵢⱼ = Hᵢ/Hⱼ, where *i* ≠ *j,* Hᵢ denotes the sequencing depth of the chromosome under test i, and Hⱼ denotes the sequencing depth of the preset chromosome j. For example, a sequencing depth sequence T₁ of chromosome 1 may be represented as T₁ = [t₁₂, t₁₃, t₁₄, ..., t₁ⱼ].

The aneuploidy of a chromosome refers to the loss or redundancy of the chromosome in the number of chromosomes relative to a normal disomy and is usually a trisomy or a monosomy.

In an exemplary embodiment, as can be known from the definition of the chromosome bin sequence and the definition of the sequencing depth sequence, the chromosome bin sequence of the chromosome under test is a fixed constant sequence; when the chromosome under test is a euploidy (disomy) in the nucleic acid sample under test, the distributions of the chromosome bin sequence and the sequencing depth sequence of the chromosome under test have no significant difference; when the chromosome under test is the aneuploidy in the nucleic acid sample under test, for example, if chromosome 21 is a trisomy, the sequencing depth of chromosome 21 becomes larger and thus the whole sequencing depth sequence T₂₁ becomes larger, if chromosome 21 is a monosomy, the sequencing depth of chromosome 21 becomes smaller and thus the whole sequencing depth sequence T₂₁ becomes smaller, and a change in the sequencing depth sequence causes a difference between the distributions of the chromosome bin sequence and the sequencing depth sequence of the chromosome under test.

The non-parametric test is used for determining whether the chromosome bin sequence and the sequencing depth sequence have a significant difference. In the presence of a significant difference, the aneuploidy detection result of the chromosome under test in the nucleic acid sample under test is the aneuploidy. In the presence of no significant difference, the aneuploidy detection result of the chromosome under test in the nucleic acid sample under test is the euploidy.

For example, the non-parametric test includes, but is not limited to, a chi-squared test, a K-S test, a Jonckheere-Terpstra test, a Mann-Whitney U test or a permutation test. The non-parametric test is not limited herein and may be customized according to the actual requirements.

Assuming that two or more chromosomes are aneuploidies in the nucleic acid sample under test, which is rare in reality, if the chromosome under test and a single preset chromosome are both aneuploidies in the nucleic acid sample under test, the overall change trend of the sequencing depth sequence may be eliminated. In this embodiment, multiple preset chromosomes are provided, that is, the sequencing depth sequence includes multiple sequencing depth parameters so that an effect of multiple aneuploid chromosomes in the nucleic acid sample under test on the overall change trend of the sequencing depth sequence can be avoided as much as possible, thereby improving the stability of the aneuploidy detection result of the chromosome and improving the accuracy of the aneuploidy detection result of the chromosome.

According to the technical solutions of this embodiment, the chromosome bin sequence built according to the human reference genome is used as a reference sequence of the chromosome under test, and the non-parametric test is performed according to the chromosome bin sequence and the sequencing depth sequence corresponding to the nucleic acid sample under test by using a correlation between a chromosome bin sequence and a sequencing depth sequence of a chromosome in nucleic acid data of euploidies so that the aneuploidy detection result of the chromosome under test in the nucleic acid sample under test is determined. The method has relatively high detection accuracy and solves the problem of dependence of the method for detecting chromosomal aneuploidy on indicator distribution in a normal sample so that a process of detecting chromosomal aneuploidy is no longer limited by a requirement for consistency between environmental parameters, and detection and maintenance costs of chromosomal aneuploidy are reduced.

In another optional embodiment, the sequencing depth parameter is a linear sequencing depth ratio, and the sequencing depth sequence determination subunit is configured to perform the operations below.

In response to the sequencing depth parameter being the linear sequencing depth ratio, at least one sequence of sequencing depth ratios corresponding to at least one euploidy sample is acquired, where each sequence of sequencing depth ratios corresponds to one respective euploidy sample and includes at least one standard sequencing depth ratio, and each standard sequencing depth ratio is the ratio of a sequencing depth of the chromosome under test in the euploidy sample to a sequencing depth of one respective preset chromosome in the euploidy sample.

A matrix of sequencing depth ratios is built according to the at least one sequence of sequencing depth ratios.

Optimization is performed according to the matrix of sequencing depth ratios and the chromosome bin sequence to obtain at least one linear fitting parameter corresponding to the chromosome under test.

A linear correction operation is performed on the at least one reference sequencing depth ratio separately according to the at least one linear fitting parameter to obtain at least one linear sequencing depth ratio.

In an exemplary embodiment, the linear sequencing depth ratio is a linear proportional relationship between the sequencing depth of the chromosome under test in the nucleic acid sample under test and the sequencing depth of the preset chromosome in the nucleic acid sample under test.

The euploidy sample is used for representing a sample where at least the chromosome under test and the at least one preset chromosome are euploidies. In this embodiment, the sequence of sequencing depth ratios includes the at least one standard sequencing depth ratio, and the standard sequencing depth ratio is the ratio of the sequencing depth of the chromosome under test in the euploidy sample to the sequencing depth of the preset chromosome in the euploidy sample.

The standard sequencing depth ratio in the sequence of sequencing depth ratios is acquired in a manner the same as or similar to a manner of acquiring the reference sequencing depth ratio in the preceding embodiment, and the details are not repeated in this embodiment.

For example, the matrix of sequencing depth ratios is an N×M matrix or an M×N matrix, where M denotes the number of euploidy samples and N denotes the number of preset chromosomes. For example, when the matrix of sequencing depth ratios is the N×M matrix, each matrix row of the matrix of sequencing depth ratios represents one sequence of sequencing depth ratios.

In an optional embodiment, after a linear depth ratio matrix is built according to the matrix of sequencing depth ratios, the method further includes: performing regularization on the matrix of sequencing depth ratios. Such setting has the following advantage: positive definiteness of the matrix of sequencing depth ratios can be ensured.

In an optional embodiment, constraints for the optimization include that an absolute value of a difference between the sequencing depth sequence and the chromosome bin sequence is minimum and that a slope parameter in each linear fitting parameter is greater than a preset positive threshold.

For example, the linear sequencing depth ratio of the chromosome under test i and the preset chromosome j may be represented as tactᵢⱼ = wᵢⱼ × tᵢⱼ + bᵢⱼ, where wᵢⱼ denotes a slope parameter corresponding to the chromosome under test *i* and the preset chromosome *j,* and bᵢⱼ denotes a constant parameter corresponding to the chromosome under test i and the preset chromosome j. Accordingly, a sum of |tactᵢⱼ-rᵢⱼ| is minimum and wᵢⱼ is greater than the preset positive threshold.

Under an ideal condition, a chromosome bin sequence of the euploidy sample is equal to a sequencing depth sequence of the euploidy sample including a reference sequencing depth ratio. However, since whole genome sequencing data are randomly and uniformly distributed, the chromosome bin sequence of the euploidy sample is positively correlated to the sequencing depth sequence of the euploidy sample including the reference sequencing depth ratio. In this embodiment, the linear correction is performed on the reference sequencing depth ratio according to the sequence of sequencing depth ratios of the euploidy sample, thereby improving the accuracy of the sequencing depth sequence and improving chromosomal aneuploidy detection performance such as sensitivity and specificity.

Based on the preceding embodiments, optionally, the aneuploidy detection result determination module 330 includes a standard test statistic determination unit, a permutation sequence group determination unit, a permutation test statistic determination unit and an aneuploidy detection result determination unit.

The standard test statistic determination unit is configured to, in response to the non-parametric test being the permutation test, determine a standard test statistic according to the chromosome bin sequence and the sequencing depth sequence, where the standard test statistic is a difference between a sequence mean of the chromosome bin sequence and a sequence mean of the sequencing depth sequence.

The permutation sequence group determination unit is configured to, according to a preset number of permutations, perform a data exchange operation on the chromosome bin sequence and the sequencing depth sequence to obtain at least one permutation sequence group, where each permutation sequence group includes a respective permuted chromosome bin sequence and a respective permuted sequencing depth sequence.

The permutation test statistic determination unit is configured to, for each permutation sequence group, determine a permutation test statistic corresponding to the permutation sequence group, where the permutation test statistic is a difference between a sequence mean of the permuted chromosome bin sequence in the permutation sequence group and a sequence mean of the permuted sequencing depth sequence in the permutation sequence group.

The aneuploidy detection result determination unit is configured to determine the aneuploidy detection result of the chromosome under test in the nucleic acid sample under test according to the standard test statistic and the permutation test statistic.

In this embodiment, the standard test statistic is the difference between the sequence mean of the chromosome bin sequence and the sequence mean of the sequencing depth sequence.

For example, the preset number of permutations may be 50,000. The preset number of permutations is not limited herein and may be customized according to the actual requirements.

In this embodiment, the permutation sequence group includes the permuted chromosome bin sequence and the permuted sequencing depth sequence, and the permutation test statistic is the difference between the sequence mean of the permuted chromosome bin sequence and the sequence mean of the permuted sequencing depth sequence.

In an optional embodiment, the aneuploidy detection result determination unit is configured to perform the operations below.

A permutation test statistic greater than the standard test statistic among at least one permutation test statistic is used as a target test statistic.

The ratio of a data volume of the target test statistic to the preset number of permutations is used as a test probability value.

In response to the test probability value being less than a significance level, the aneuploidy detection result of the chromosome under test in the nucleic acid sample under test is determined to be the aneuploidy.

In response to the test probability value being greater than or equal to the significance level, the aneuploidy detection result of the chromosome under test in the nucleic acid sample under test is determined to be the euploidy.

For example, the significance level may be 0.01 or 0.001. The significance level is not limited herein and may be customized according to the actual requirements.

In an exemplary embodiment, it is assumed that a null hypothesis H0 is established that the distributions of the chromosome bin sequence and the sequencing depth sequence have no difference, that is, the chromosome under test is the euploidy in the nucleic acid sample under test; and it is assumed that an alternative hypothesis H1 is established that the distributions of the chromosome bin sequence and the sequencing depth sequence have a difference, that is, the chromosome under test is the aneuploidy in the nucleic acid sample under test. If the test probability value P is less than the significance level, the null hypothesis H0 is rejected, that is, the aneuploidy detection result of the chromosome under test in the nucleic acid sample under test is determined to be the aneuploidy. If the test probability value P is greater than or equal to the significance level, the null hypothesis H0 is accepted, that is, the aneuploidy detection result of the chromosome under test in the nucleic acid sample under test is determined to be the euploidy.

The apparatus for detecting chromosomal aneuploidy according to the embodiment of the present invention may perform the method for detecting chromosomal aneuploidy according to any embodiment of the present invention and has function modules and beneficial effects corresponding to the performed method.

FIG. 5 is a structure diagram of an electronic device according to an embodiment of the present invention. An electronic device 10 is intended to represent various forms of digital computers, for example, a laptop computer, a desktop computer, a worktable, a server, a blade server, a mainframe computer and an applicable computer. The electronic device may also represent various forms of mobile apparatuses, for example, a personal digital assistant, a cellphone, a smartphone, a wearable device (such as a helmet, glasses or a watch) and a similar computing apparatus. Herein the shown components, the connections and relationships between these components and the functions of these components are illustrative and are not intended to limit the implementation of the present invention as described and/or claimed herein.

As shown in FIG. 5, the electronic device 10 includes at least one processor 11 and a memory communicatively connected to the at least one processor 11, such as a read-only memory (ROM) 12 or a random-access memory (RAM) 13. The memory stores a computer program executable by the at least one processor 11. The processor 11 can perform various appropriate actions and processing according to a computer program stored in the ROM 12 or a computer program loaded into the RAM 13 from a storage unit 18. Various programs and data required for the operation of the electronic device 10 may also be stored in the RAM 13. The processor 11, the ROM 12 and the RAM 13 are connected to each other through a bus 14. An input/output (I/O) interface 15 is also connected to the bus 14.

Multiple components in the electronic device 10 are connected to the I/O interface 15. The multiple components include an input unit 16 such as a keyboard or a mouse, an output unit 17 such as various types of displays or speakers, the storage unit 18 such as a magnetic disk or an optical disk, and a communication unit 19 such as a network card, a modem or a wireless communication transceiver. The communication unit 19 allows the electronic device 10 to exchange information or data with other devices over a computer network such as the Internet and/or various telecommunications networks.

The processor 11 may be various general-purpose and/or special-purpose processing components having processing and computing capabilities. Examples of the processor 11 include, but are not limited to, a central processing unit (CPU), a graphics processing unit (GPU), a special-purpose artificial intelligence (AI) computing chip, a processor executing machine learning models and algorithms, a digital signal processor (DSP) and any appropriate processor, controller and microcontroller. The processor 11 performs the preceding methods and processing, such as the method for detecting chromosomal aneuploidy according to the preceding embodiments.

In some embodiments, the method for detecting chromosomal aneuploidy according to the preceding embodiments may be implemented as a computer program tangibly included in a computer-readable storage medium such as the storage unit 18. In some embodiments, part or all of computer programs may be loaded and/or installed onto the electronic device 10 via the ROM 12 and/or the communication unit 19. When the computer programs are loaded into the RAM 13 and executed by the processor 11, one or more steps of the preceding method for detecting chromosomal aneuploidy may be performed. Alternatively, in other embodiments, the processor 11 may be configured in any other appropriate manner (for example, by means of firmware) to perform the method for detecting chromosomal aneuploidy.

Herein various embodiments of the preceding systems and techniques may be implemented in the following systems or a combination thereof: digital electronic circuitry, integrated circuitry, field-programmable gate arrays (FPGAs), application-specific integrated circuits (ASICs), application-specific standard products (ASSPs), systems on chips (SoCs), complex programmable logic devices (CPLDs), computer hardware, firmware, software and/or combinations thereof. The various embodiments may include implementations in one or more computer programs. The one or more computer programs are executable and/or interpretable on a programmable system including at least one programmable processor. The programmable processor may be a special-purpose or general-purpose programmable processor for receiving data and instructions from a memory system, at least one input apparatus and at least one output apparatus and transmitting data and instructions to the memory system, the at least one input apparatus and the at least one output apparatus.

Computer programs for implementation of the method for detecting chromosomal aneuploidy of the present invention may be written in one programming language or any combination of multiple programming languages. The computer programs may be provided for a processor of a general-purpose computer, a special-purpose computer or another programmable data processing apparatus to enable functions/operations specified in a flowchart and/or a block diagram to be implemented when the computer programs are executed by the processor. The computer programs may be executed entirely on a machine, partly on a machine, as a stand-alone software package, partly on a machine and partly on a remote machine, or entirely on a remote machine or a server.

In the context of the present application, the computer-readable storage medium may be a tangible medium that may include or store a computer program for use by or in connection with an instruction execution system, apparatus or device. The computer-readable storage medium may include, but is not limited to, an electronic, magnetic, optical, electromagnetic, infrared or semiconductor system, apparatus or device or any appropriate combination thereof. Alternatively, the computer-readable storage medium may be a machine-readable storage medium. Examples of the machine-readable storage medium include an electrical connection based on at least one wire, a portable computer disk, a hard disk, a RAM, a ROM, an erasable programmable read-only memory (EPROM), a flash memory, an optical fiber, a portable compact disc read-only memory (CD-ROM), an optical storage device, a magnetic storage device or any appropriate combination thereof.

In order that interaction with a user is provided, the systems and techniques described herein may be implemented on a terminal device. The terminal device has a display apparatus (for example, a cathode-ray tube (CRT) or a liquid-crystal display (LCD) monitor) for displaying information to the user; and a keyboard and a pointing apparatus (for example, a mouse or a trackball) through which the user can provide input for the terminal device. Other types of apparatuses may also provide interaction with a user. For example, feedback provided for the user may be sensory feedback in any form (for example, visual feedback, auditory feedback or tactile feedback); and input from the user may be received in any form (including acoustic input, voice

The systems and techniques described herein may be implemented in a computing system including a back-end component (for example, a data server), a computing system including a middleware component (for example, an application server), a computing system including a front-end component (for example, a client computer having a graphical user interface or a web browser through which a user can interact with embodiments of the systems and techniques described herein) or a computing system including any combination of such back-end, middleware or front-end components. Components of a system may be interconnected by any form or medium of digital data communication (for example, a communication network). Examples of the communication network include a local area network (LAN), a wide area network (WAN), a blockchain network and the Internet.

The computing system may include clients and servers. A client and a server are generally remote from each other and typically interact through a communication network. The relationship between the client and the server arises by virtue of computer programs running on respective computers and having a client-server relationship to each other. The server may be a cloud server, also referred to as a cloud computing server or a cloud host. As a host product in a cloud computing service system, the server solves the defects of difficult management and weak service scalability in conventional physical host and virtual private server (VPS) services.

It is to be understood that various forms of the preceding flows may be used, with steps reordered, added or removed. For example, the steps described in the present invention may be performed in parallel, in sequence or in a different order as long as the desired results of the technical solutions of the present invention can be achieved. The execution sequence of these steps is not limited herein.

## Claims

1. A method for detecting chromosomal aneuploidy, comprising:
determining a chromosome bin sequence Rᵢ of a chromosome i under test according to reference genome nucleic acid data of a human reference genome, wherein the chromosome bin sequence Rᵢ comprises at least one bin number ratio rᵢⱼ, and each of the at least one bin number ratio is a ratio rᵢⱼ= Lᵢ/Lⱼ, where *i* ≠ *j,* of a number Lᵢ of nucleic acid bins of the chromosome *i* under test for aneuploidy in the human reference genome to a number Lⱼ of nucleic acid bins of a respective one of at least one preset chromosome j not under test for aneuploidy in the human reference genome;
determining a sequencing depth sequence of the chromosome i under test according to whole genome sequencing data of a nucleic acid sample under test, wherein the sequencing depth sequence comprises at least one sequencing depth parameter, and each of the at least one sequencing depth parameter is either a reference sequencing depth ratio tᵢⱼ = Hᵢ/Hⱼ, where i ≠ j, of a sequencing depth Hᵢ of the chromosome i under test in the nucleic acid sample under test to a sequencing depth Hⱼ of a respective one of the at least one preset chromosome j not under test in the nucleic acid sample under test, or a linear sequencing depth ratio which is based on the reference sequencing depth ratio;
according to the chromosome bin sequence and the sequencing depth sequence, performing a non-parametric test to determine an aneuploidy detection result of the chromosome under test in the nucleic acid sample under test

2. The method according to claim 1, wherein determining the chromosome bin sequence of the chromosome under test according to the reference genome nucleic acid data of the human reference genome comprises:
acquiring, from the reference genome nucleic acid data, a reference chromosome nucleic acid datum of the chromosome under test and a reference chromosome nucleic acid datum of each of the at least one preset chromosome;
for each reference chromosome nucleic acid datum, performing bin division on the reference chromosome nucleic acid datum according to a bin division rule, and according to a bin division result, determining the number of nucleic acid bins of the chromosome under test and a number of nucleic acid bins of each of the at least one preset chromosome; and
determining the chromosome bin sequence of the chromosome under test according to the number of nucleic acid bins of the chromosome under test and the number of nucleic acid bins of each of the at least one preset chromosome.

3. The method according to claim 2, wherein determining, according to the bin division result, the number of nucleic acid bins of the chromosome under test and the number of nucleic acid bins of each of the at least one preset chromosome comprises:
performing a deletion operation on a nucleic acid bin not comprising any known bases in the bin division result; and
counting remaining nucleic acid bins in the bin division result after the deletion operation to obtain the number of nucleic acid bins of the chromosome under test and the number of nucleic acid bins of each of the at least one preset chromosome.

4. The method according to claim 2, wherein determining the sequencing depth sequence of the chromosome under test according to the whole genome sequencing data of the nucleic acid sample under test comprises:
acquiring, from the whole genome sequencing data, a chromosome sequencing datum of the chromosome under test and a chromosome sequencing datum of each of the at least one preset chromosome;
for each chromosome sequencing datum, performing sequence alignment on the chromosome sequencing datum and at least one nucleic acid bin of a respective chromosome, determining a number of nucleic acid sequences in an alignment datum of each of the at least one nucleic acid bin, and using the number of nucleic acid sequences in alignment data of the at least one nucleic acid bin as a sequencing depth of the respective chromosome; and
determining the sequencing depth sequence of the chromosome under test according to the sequencing depth of the chromosome under test and a sequencing depth of each of the at least one preset chromosome.

5. The method according to claim 4, wherein determining the number of nucleic acid sequences in the alignment datum of each of the at least one nucleic acid bin comprises:
acquiring an initial number of sequences in the alignment datum of each of the at least one nucleic acid bin; and
performing a correction operation on the initial number of sequences to obtain the number of nucleic acid sequences in the alignment datum of each of the at least one nucleic acid bin.

6. The method according to claim 5, wherein the correction operation comprises at least one of effective base length correction, outlier correction, mappability correction or guanine-cytosine (GC)-content correction.

7. The method according to claim 1, wherein determining the sequencing depth sequence of the chromosome under test according to the at least one reference sequencing depth ratio comprises:
in response to the at least one sequencing depth parameter being the at least one linear sequencing depth ratio, acquiring at least one sequence of sequencing depth ratios corresponding to at least one euploidy sample, wherein each of the at least one sequence of sequencing depth ratios comprises at least one standard sequencing depth ratio, and for each sequence of sequencing depth ratios, the sequence of sequencing depth ratios corresponds to a respective one of the at least one euploidy sample, each of the at least one standard sequencing depth ratio in the sequence of sequencing depth ratios corresponds to a respective one of the at least one preset chromosome, and the standard sequencing depth ratio is a ratio of a sequencing depth of the chromosome under test to a sequencing depth of the respective preset chromosome in the respective euploidy sample;
building a matrix of sequencing depth ratios according to the at least one sequence of sequencing depth ratios;
performing optimization according to the matrix of sequencing depth ratios and the chromosome bin sequence to obtain at least one linear fitting parameter corresponding to the chromosome under test; and
performing a linear correction operation on the at least one reference sequencing depth ratio separately according to the at least one linear fitting parameter to obtain the at least one linear sequencing depth ratio.

8. The method according to claim 7, wherein constraints for the optimization comprise that an absolute value of a difference between the sequencing depth sequence and the chromosome bin sequence is minimum and that each of the at least one linear fitting parameter comprises a slope which is greater than a preset positive threshold.

9. The method according to claim 8, wherein the linear sequencing depth ratio of the chromosome under test *i* and the preset chromosome *j* is represented as tactᵢⱼ = wᵢⱼ × tᵢⱼ + bᵢⱼ, where wᵢⱼ denotes a slope corresponding to the chromosome under test *i* and the preset chromosome *j,* and bᵢⱼ denotes a constant parameter corresponding to the chromosome under test *i* and the preset chromosome *j;*
a sum of |tactᵢⱼ-rᵢⱼ| is minimum and wᵢⱼ is greater than the preset positive threshold.

10. The method according to any one of claims 1 to 9, wherein according to the chromosome bin sequence and the sequencing depth sequence, performing the non-parametric test to determine the aneuploidy detection result of the chromosome under test in the nucleic acid sample under test comprises:
in response to the non-parametric test being a permutation test, determining a standard test statistic according to the chromosome bin sequence and the sequencing depth sequence, wherein the standard test statistic is a difference between a sequence mean of the chromosome bin sequence and a sequence mean of the sequencing depth sequence;
according to a preset number of permutations, performing a data exchange operation on the chromosome bin sequence and the sequencing depth sequence to obtain at least one permutation sequence group, wherein each of the at least one permutation sequence group comprises a respective permuted chromosome bin sequence and a respective permuted sequencing depth sequence;
for each permutation sequence group, determining a permutation test statistic corresponding to the permutation sequence group, wherein the permutation test statistic is a difference between a sequence mean of the permuted chromosome bin sequence in the permutation sequence group and a sequence mean of the permuted sequencing depth sequence in the permutation sequence group; and
determining the aneuploidy detection result of the chromosome under test in the nucleic acid sample under test according to the standard test statistic and the permutation test statistic.

11. The method according to claim 10, wherein determining the aneuploidy detection result of the chromosome under test in the nucleic acid sample under test according to the standard test statistic and at least one permutation test statistic comprises:
using a permutation test statistic greater than the standard test statistic among the at least one permutation test statistic as a target test statistic;
using a ratio of a data volume of the target test statistic to the preset number of permutations as a test probability value;
in response to the test probability value being less than a significance level, determining the aneuploidy detection result of the chromosome under test in the nucleic acid sample under test to be an aneuploidy; and
in response to the test probability value being greater than or equal to the significance level, determining the aneuploidy detection result of the chromosome under test in the nucleic acid sample under test to be a euploidy.

12. The method according to any one of claims 1 to 9, further comprising:
extracting a free nucleic acid from the nucleic acid sample under test;
performing polymerase chain reaction (PCR) amplification on the free nucleic acid and performing sample pretreatment to obtain a nucleic acid library; and
performing whole genome sequencing on the nucleic acid library to obtain the whole genome sequencing data of the nucleic acid sample under test.

13. An apparatus for detecting chromosomal aneuploidy, comprising:
a chromosome bin sequence determination module, which is configured to determine a chromosome bin sequence of a chromosome under test according to reference genome nucleic acid data of a human reference genome, wherein the chromosome bin sequence comprises at least one bin number ratio rᵢⱼ, and each of the at least one bin number ratio is a ratio rᵢⱼ= Lᵢ/Lⱼ, where i ≠ *j,* of a number Lᵢ of nucleic acid bins of the chromosome *i* under test for aneuploidy in the human reference genome to a number Lⱼ of nucleic acid bins of a respective one of at least one preset chromosome j not under test for aneuploidy in the human reference genome;
a sequencing depth sequence determination module, which is configured to determine a sequencing depth sequence of the chromosome i under test according to whole genome sequencing data of a nucleic acid sample under test, wherein the sequencing depth sequence comprises at least one sequencing depth parameter, and each of the at least one sequencing depth parameter is either a reference sequencing depth ratio tᵢⱼ = Hᵢ/Hⱼ, where i ≠ j, of a sequencing depth Hᵢ of the chromosome i under test in the nucleic acid sample under test to a sequencing depth Hⱼ of a respective one of the at least one preset chromosome j not under test in the nucleic acid sample under test, or a linear sequencing depth ratio which is based on the reference sequencing depth ratio; and
an aneuploidy detection result determination module, which is configured to, according to the chromosome bin sequence and the sequencing depth sequence, perform a non-parametric test to obtain an aneuploidy detection result of the chromosome under test in the nucleic acid sample under test.

14. A computer-readable storage medium for storing a computer instruction, wherein the computer instruction, when executed by a processor, causes the processor to perform the method for detecting chromosomal aneuploidy according to any one of claims 1 to 12.

## Patentansprüche

1. Verfahren zum Nachweisen chromosomaler Aneuploidie, umfassend:
Bestimmen einer Chromosomen-Bin-Sequenz Rᵢ eines zu testenden Chromosoms *i* gemäß Referenzgenomnukleinsäuredaten eines menschlichen Referenzgenoms, wobei die Chromosomen-Bin-Sequenz Rᵢ mindestens ein Bin-Anzahlverhältnis rᵢⱼ umfasst, und jedes des mindestens einen Bin-Anzahlverhältnisses ein Verhältnis rᵢⱼ= Lᵢ/Lⱼ ist, wobei *i ≠ j,* einer Anzahl Lᵢ von Nukleinsäure-Bins des zu testenden Chromosoms *i* auf Aneuploidie in dem menschlichen Referenzgenom zu einer Anzahl Lⱼ von Nukleinsäure-Bins eines jeweiligen einen des mindestens einen voreingestellten, nicht auf Aneuploidie zu testenden Chromosoms *j*, in dem menschlichen Referenzgenom ist;
Bestimmen einer Sequenzierungstiefensequenz des zu testenden Chromosoms *i* gemäß Gesamtgenomsequenzierungsdaten einer zu testenden Nukleinsäureprobe, wobei die Sequenzierungstiefensequenz mindestens einen Sequenzierungstiefenparameter umfasst und jeder des mindestens einen Sequenzierungstiefenparameters entweder ein Referenzsequenzierungstiefenverhältnis tᵢⱼ = Hᵢ/Hⱼ, wobei i ≠ j, einer Sequenzierungstiefe Hᵢ des zu testenden Chromosoms i in der zu testenden Nukleinsäureprobe zu einer Sequenzierungstiefe Hⱼ eines jeweiligen einen des mindestens einen voreingestellten, nicht zu testenden Chromosoms *j* in der zu testenden Nukleinsäureprobe, oder ein lineares Sequenzierungstiefenverhältnis, das auf dem Referenzsequenzierungstiefenverhältnis basiert, ist;
gemäß der Chromosomen-Bin-Sequenz und der Sequenzierungstiefensequenz Durchführen eines nicht parametrischen Tests, um ein Aneuploidienachweisergebnis des zu testenden Chromosoms in der zu testenden Nukleinsäureprobe zu bestimmen.

2. Verfahren nach Anspruch 1, wobei das Bestimmen der Chromosomen-Bin-Sequenz des zu testenden Chromosoms gemäß den Referenzgenomnukleinsäuredaten des menschlichen Referenzgenoms umfasst:
Erfassen, aus den Referenzgenomnukleinsäuredaten, eines Referenzchromosomnukleinsäuredatums des zu testenden Chromosoms und eines Referenzchromosomnukleinsäuredatums jedes des mindestens einen voreingestellten Chromosoms;
für jedes Referenzchromosomnukleinsäuredatum Durchführen einer Bin-Unterteilung an dem Referenzchromosomnukleinsäuredatum gemäß einer Bin-Unterteilungsregel, und gemäß einem Bin-Unterteilungsergebnis, Bestimmen der Anzahl der Nukleinsäure-Bins des zu testenden Chromosoms und einer Anzahl der Nukleinsäure-Bins jedes des mindestens einen voreingestellten Chromosoms; und
Bestimmen der Chromosomen-Bin-Sequenz des zu testenden Chromosoms gemäß der Anzahl der Nukleinsäure-Bins des zu testenden Chromosoms und der Anzahl der Nukleinsäure-Bins jedes des mindestens einen voreingestellten Chromosoms.

3. Verfahren nach Anspruch 2, wobei das Bestimmen, gemäß dem Bin-Unterteilungsergebnis, der Anzahl der Nukleinsäure-Bins des zu testenden Chromosoms und der Anzahl der Nukleinsäure-Bins jedes des mindestens einen voreingestellten Chromosoms umfasst:
Durchführen einer Löschoperation an einem Nukleinsäure-Bin, umfassend keine bekannten Basen in dem Bin-Unterteilungsergebnis; und
Zählen verbleibender Nukleinsäure-Bins in dem Bin-Unterteilungsergebnis nach der Löschoperation, um die Anzahl der Nukleinsäure-Bins des zu testenden Chromosoms und die Anzahl der Nukleinsäure-Bins jedes des mindestens einen voreingestellten Chromosoms zu erhalten.

4. Verfahren nach Anspruch 2, wobei das Bestimmen der Sequenzierungstiefensequenz des zu testenden Chromosoms gemäß den Gesamtgenomsequenzierungsdaten der zu testenden Nukleinsäureprobe umfasst:
Erfassen, aus den Ganzgenomsequenzierungsdaten, eines Chromosomensequenzierungsdatums des zu testenden Chromosoms und eines Chromosomensequenzierungsdatums jedes des mindestens einen voreingestellten Chromosoms;
für jedes Chromosomensequenzierungsdatum Durchführen einer Sequenzausrichtung an dem Chromosomensequenzierungsdatum und mindestens einem Nukleinsäure-Bin eines jeweiligen Chromosoms, Bestimmen einer Anzahl der Nukleinsäuresequenzen in einem Ausrichtungsdatum jedes des mindestens einen Nukleinsäure-Bins und Verwenden der Anzahl der Nukleinsäuresequenzen in Ausrichtungsdaten des mindestens einen Nukleinsäure-Bins als eine Sequenzierungstiefe des jeweiligen Chromosoms; und
Bestimmen der Sequenzierungstiefensequenz des zu testenden Chromosoms gemäß der Sequenzierungstiefe des zu testenden Chromosoms und einer Sequenzierungstiefe jedes des mindestens einen voreingestellten Chromosoms.

5. Verfahren nach Anspruch 4, wobei das Bestimmen der Anzahl der Nukleinsäuresequenzen in dem Ausrichtungsdatum jedes des mindestens einen Nukleinsäure-Bins umfasst:
Erfassen einer anfänglichen Anzahl von Sequenzen in dem Ausrichtungsdatum jedes des mindestens einen Nukleinsäure-Bins; und
Durchführen einer Korrekturoperation an der anfänglichen Anzahl von Sequenzen, um die Anzahl der Nukleinsäuresequenzen in dem Ausrichtungsdatum jedes des mindestens einen Nukleinsäure-Bins zu erhalten.

6. Verfahren nach Anspruch 5, wobei die Korrekturoperation mindestens eine von einer effektiven Basenlängenkorrektur, einer Ausreißerkorrektur, einer Kartierbarkeitskorrektur oder einer Guanin-Cytosin-Gehaltskorrektur (GC-Gehaltskorrektur) umfasst.

7. Verfahren nach Anspruch 1, wobei das Bestimmen der Sequenzierungstiefensequenz des zu testenden Chromosoms gemäß dem mindestens einen Referenzsequenzierungstiefenverhältnis umfasst:
als Reaktion darauf, dass der mindestens eine Sequenzierungstiefenparameter das mindestens eine lineare Sequenzierungstiefenverhältnis ist, Erfassen mindestens einer Sequenz von Sequenzierungstiefenverhältnissen, die mindestens einer Euploidieprobe entspricht, wobei jede der mindestens einen Sequenz von Sequenzierungstiefenverhältnissen mindestens ein Standardsequenzierungstiefenverhältnis umfasst und für jede Sequenz von Sequenzierungstiefenverhältnissen die Sequenz von Sequenzierungstiefenverhältnissen einer jeweiligen einen der mindestens einen Euploidieprobe entspricht, jedes des mindestens einen Standardsequenzierungstiefenverhältnisses in der Sequenz von Sequenzierungstiefenverhältnissen einem jeweiligen einen des mindestens einen voreingestellten Chromosoms entspricht und das Standardsequenzierungstiefenverhältnis ein Verhältnis einer Sequenzierungstiefe des zu testenden Chromosoms zu einer Sequenzierungstiefe des jeweiligen voreingestellten Chromosoms in der jeweiligen Euploidieprobe ist;
Erstellen einer Matrix von Sequenzierungstiefenverhältnissen gemäß der mindestens einen Sequenz von Sequenzierungstiefenverhältnissen;
Durchführen einer Optimierung gemäß der Matrix von Sequenzierungstiefenverhältnissen und der Chromosomen-Bin-Sequenz, um mindestens einen linearen Anpassungsparameter, der dem zu testenden Chromosom entspricht, zu erhalten; und
Durchführen einer linearen Korrekturoperation an dem mindestens einen Referenzsequenzierungstiefenverhältnis getrennt gemäß dem mindestens einen linearen Anpassungsparameter, um das mindestens eine lineare Sequenzierungstiefenverhältnis zu erhalten.

8. Verfahren nach Anspruch 7, wobei Grenzbedingungen für die Optimierung umfassen, dass ein absoluter Wert einer Differenz zwischen der Sequenzierungstiefensequenz und der Chromosomen-Bin-Sequenz minimal ist und dass jeder des mindestens einen linearen Anpassungsparameters eine Steigung, die größer als eine voreingestellte positive Schwelle ist, umfasst.

9. Verfahren nach Anspruch 8, wobei das lineare Sequenzierungstiefenverhältnis des zu testenden Chromosoms *i* und des voreingestellten Chromosoms *j* als tactᵢⱼ = wᵢⱼ× tᵢⱼ + bᵢⱼ dargestellt wird, wobei wᵢⱼ eine Steigung, die dem zu testenden Chromosom *i* und dem voreingestellten Chromosom *j* entspricht, bezeichnet und bᵢⱼ einen konstanten Parameter, der dem zu testenden Chromosom *i* und dem voreingestellten Chromosom *j* entspricht, bezeichnet;
eine Summe von |tactᵢⱼ-rᵢⱼ| minimal ist und wᵢⱼ größer als die voreingestellte positive Schwelle ist.

10. Verfahren nach einem der Ansprüche 1 bis 9, wobei, gemäß der Chromosomen-Bin-Sequenz und der Sequenzierungstiefensequenz, das Durchführen des nicht parametrischen Tests, um das Aneuploidienachweisergebnis des zu testenden Chromosoms in der zu testenden Nukleinsäureprobe zu bestimmen, umfasst:
als Reaktion darauf, dass der nicht parametrische Test ein Permutationstest ist, Bestimmen einer Standardteststatistik gemäß der Chromosomen-Bin-Sequenz und der Sequenzierungstiefensequenz, wobei die Standardteststatistik eine Differenz zwischen einem Sequenzmittelwert der Chromosomen-Bin-Sequenz und einem Sequenzmittelwert der Sequenzierungstiefensequenz ist;
gemäß einer voreingestellten Anzahl von Permutationen Durchführen einer Datenaustauschoperation an der Chromosomen-Bin-Sequenz und der Sequenzierungstiefensequenz, um mindestens eine Permutationssequenzgruppe zu erhalten, wobei jede der mindestens einen Permutationssequenzgruppe eine jeweilige permutierte Chromosomen-Bin-Sequenz und eine jeweilige permutierte Sequenzierungstiefensequenz umfasst;
für jede Permutationssequenzgruppe Bestimmen einer Permutationsteststatistik, die der Permutationssequenzgruppe entspricht, wobei die Permutationsteststatistik eine Differenz zwischen einem Sequenzmittelwert der permutierten Chromosomen-Bin-Sequenz in der Permutationssequenzgruppe und einem Sequenzmittelwert der permutierten Sequenzierungstiefensequenz in der Permutationssequenzgruppe ist; und
Bestimmen des Aneuploidienachweisergebnisses des zu testenden Chromosoms in der zu testenden Nukleinsäureprobe gemäß der Standardteststatistik und der Permutationsteststatistik.

11. Verfahren nach Anspruch 10, wobei das Bestimmen des Aneuploidienachweisergebnisses des zu testenden Chromosoms in der zu testenden Nukleinsäureprobe gemäß der Standardteststatistik und mindestens einer Permutationsteststatistik umfasst:
Verwenden einer Permutationsteststatistik, die unter der mindestens einen Permutationsteststatistik größer als die Standardteststatistik ist, als eine Zielteststatistik;
Verwenden eines Verhältnisses eines Datenvolumens der Zielteststatistik zu der voreingestellten Anzahl von Permutationen als einen Testwahrscheinlichkeitswert;
als Reaktion darauf, dass der Testwahrscheinlichkeitswert kleiner als ein Signifikanzniveau ist, Bestimmen des Aneuploidienachweisergebnisses des zu testenden Chromosoms in der zu testenden Nukleinsäureprobe als eine Aneuploidie; und
als Reaktion darauf, dass der Testwahrscheinlichkeitswert größer als oder gleich dem Signifikanzniveau ist, Bestimmen des Aneuploidienachweisergebnisses des zu testenden Chromosoms in der zu testenden Nukleinsäureprobe als eine Euploidie.

12. Verfahren nach einem der Ansprüche 1 bis 9, ferner umfassend:
Extrahieren einer freien Nukleinsäure aus der zu testenden Nukleinsäureprobe;
Durchführen einer Polymerasekettenreaktionsamplifikation (PCR-Amplifikation) an der freien Nukleinsäure und Durchführen von Probenvorbehandlung, um eine Nukleinsäurebibliothek zu erhalten; und
Durchführen einer Gesamtgenomsequenzierung an der Nukleinsäurebibliothek, um die Gesamtgenomsequenzierungsdaten der zu testenden Nukleinsäureprobe zu erhalten.

13. Einrichtung zum Nachweisen chromosomaler Aneuploidie, umfassend:
ein Chromosomen-Bin-Sequenzbestimmungsmodul, das konfiguriert ist, um eine Chromosomen-Bin-Sequenz eines zu testenden Chromosoms gemäß Referenzgenomnukleinsäuredaten eines menschlichen Referenzgenoms zu bestimmen, wobei die Chromosomen-Bin-Sequenz mindestens ein Bin-Anzahlverhältnis rᵢⱼ umfasst, und jedes des mindestens einen Bin-Anzahlverhältnisses ein Verhältnis rᵢⱼ= Lᵢ/Lⱼ ist, wobei *i ≠ j,* einer Anzahl Lᵢ von Nukleinsäure-Bins des zu testenden Chromosoms *i* auf Aneuploidie in dem menschlichen Referenzgenom zu einer Anzahl Lⱼ von Nukleinsäure-Bins eines jeweiligen einen des mindestens einen voreingestellten, nicht auf Aneuploidie zu testenden Chromosoms *j*, in dem menschlichen Referenzgenom ist;
ein Sequenzierungstiefensequenzbestimmungsmodul, das konfiguriert ist, um eine Sequenzierungstiefensequenz des zu testenden Chromosoms *i* gemäß Gesamtgenomsequenzierungsdaten einer zu testenden Nukleinsäureprobe zu bestimmen, wobei die Sequenzierungstiefensequenz mindestens einen Sequenzierungstiefenparameter umfasst und jeder des mindestens einen Sequenzierungstiefenparameters entweder ein Referenzsequenzierungstiefenverhältnis tᵢⱼ = Hᵢ/Hⱼ, wobei i ≠ j, einer Sequenzierungstiefe Hᵢ des zu testenden Chromosoms i in der zu testenden Nukleinsäureprobe zu einer Sequenzierungstiefe Hⱼ eines jeweiligen einen des mindestens einen voreingestellten, nicht zu testenden Chromosoms *j* in der zu testenden Nukleinsäureprobe, oder ein lineares Sequenzierungstiefenverhältnis, das auf dem Referenzsequenzierungstiefenverhältnis basiert, ist; und
ein Aneuploidienachweisergebnisbestimmungsmodul, das konfiguriert ist, um, gemäß der Chromosomen-Bin-Sequenz und der Sequenzierungstiefensequenz, einen nicht parametrischen Test durchzuführen, um ein Aneuploidienachweisergebnis des zu testenden Chromosoms in der zu testenden Nukleinsäureprobe zu erhalten.

14. Computerlesbares Speichermedium zum Speichern einer Computeranweisung, wobei die Computeranweisung, wenn sie durch einen Prozessor ausgeführt wird, den Prozessor veranlasst, das Verfahren zum Nachweisen chromosomaler Aneuploidie nach einem der Ansprüche 1 bis 12 durchzuführen.

## Revendications

1. Procédé permettant de détecter une aneuploïdie chromosomique, comprenant :
la détermination d'une séquence de compartiments de chromosome Rᵢ d'un chromosome *i* en cours de test selon des données d'acide nucléique de génome de référence d'un génome de référence humain, dans lequel la séquence de compartiments de chromosome Rᵢ comprend au moins un rapport de nombre de compartiments rᵢⱼ, et chacun de l'au moins un rapport de nombre de compartiments est un rapport rᵢⱼ = Lᵢ/Lⱼ, où *i ≠ j,* entre un nombre Lᵢ de compartiments d'acide nucléique du chromosome *i* en cours de test pour une aneuploïdie dans le génome de référence humain et un nombre Lⱼ de compartiments d'acide nucléique d'un chromosome respectif d'au moins un chromosome *j* prédéfini non en cours de test pour une aneuploïdie dans le génome de référence humain ;
la détermination d'une séquence de profondeur de séquençage du chromosome *i* en cours de test selon des données de séquençage de génome entier d'un échantillon d'acide nucléique en cours de test, dans lequel la séquence de profondeur de séquençage comprend au moins un paramètre de profondeur de séquençage, et chacun de l'au moins un paramètre de profondeur de séquençage est soit un rapport de profondeur de séquençage de référence tᵢⱼ = Hᵢ/Hⱼ, où i ≠ j, entre une profondeur de séquençage Hᵢ du chromosome i en cours de test dans l'échantillon d'acide nucléique en cours de test et une profondeur de séquençage Hⱼ d'un chromosome respectif de l'au moins un chromosome *j* prédéfini non en cours de test dans l'échantillon d'acide nucléique en cours de test, soit un rapport de profondeur de séquençage linéaire qui est basé sur le rapport de profondeur de séquençage de référence ;
selon la séquence de compartiments de chromosome et la séquence de profondeur de séquençage, la réalisation d'un test non paramétrique pour déterminer un résultat de détection d'aneuploïdie du chromosome en cours de test dans l'échantillon d'acide nucléique en cours de test

2. Procédé selon la revendication 1, dans lequel la détermination de la séquence de compartiments de chromosome du chromosome en cours de test selon les données d'acide nucléique de génome de référence du génome de référence humain, comprend :
l'acquisition, à partir des données d'acide nucléique de génome de référence, d'une donnée d'acide nucléique de chromosome de référence du chromosome en cours de test et d'une donnée d'acide nucléique de chromosome de référence de chacun de l'au moins un chromosome prédéfini ;
pour chaque donnée d'acide nucléique de chromosome de référence, la réalisation d'une division en compartiments sur la donnée d'acide nucléique de chromosome de référence selon une règle de division en compartiments, et selon un résultat de division en compartiments, la détermination du nombre de compartiments d'acide nucléique du chromosome en cours de test et d'un nombre de compartiments d'acide nucléique de chacun de l'au moins un chromosome prédéfini ; et
la détermination de la séquence de compartiments de chromosome du chromosome en cours de test selon le nombre de compartiments d'acide nucléique du chromosome en cours de test et le nombre de compartiments d'acide nucléique de chacun de l'au moins un chromosome prédéfini.

3. Procédé selon la revendication 2, dans lequel la détermination, selon le résultat de division en compartiments, du nombre de compartiments d'acide nucléique du chromosome en cours de test et du nombre de compartiments d'acide nucléique de chacun de l'au moins un chromosome prédéfini, comprend :
la réalisation d'une opération de délétion sur un compartiment d'acide nucléique ne comprenant aucune base connue dans le résultat de division en compartiments ; et
le comptage de compartiments d'acide nucléique restants dans le résultat de division en compartiments après l'opération de délétion pour obtenir le nombre de compartiments d'acide nucléique du chromosome en cours de test et le nombre de compartiments d'acide nucléique de chacun de l'au moins un chromosome prédéfini.

4. Procédé selon la revendication 2, dans lequel la détermination de la séquence de profondeur de séquençage du chromosome en cours de test selon les données de séquençage de génome entier de l'échantillon d'acide nucléique en cours de test, comprend :
l'acquisition, à partir des données de séquençage de génome entier, d'une donnée de séquençage de chromosome du chromosome en cours de test et d'une donnée de séquençage de chromosome de chacun de l'au moins un chromosome prédéfini ;
pour chaque donnée de séquençage de chromosome, la réalisation d'un alignement de séquence sur la donnée de séquençage de chromosome et au moins un compartiment d'acide nucléique d'un chromosome respectif, la détermination d'un nombre de séquences d'acide nucléique dans une donnée d'alignement de chacun de l'au moins un compartiment d'acide nucléique, et l'utilisation du nombre de séquences d'acide nucléique dans des données d'alignement de l'au moins un compartiment d'acide nucléique en tant que profondeur de séquençage du chromosome respectif ; et
la détermination de la séquence de profondeur de séquençage du chromosome en cours de test selon la profondeur de séquençage du chromosome en cours de test et une profondeur de séquençage de chacun de l'au moins un chromosome prédéfini.

5. Procédé selon la revendication 4, dans lequel la détermination du nombre de séquences d'acide nucléique dans la donnée d'alignement de chacun de l'au moins un compartiment d'acide nucléique, comprend :
l'acquisition d'un nombre initial de séquences dans la donnée d'alignement de chacun de l'au moins un compartiment d'acide nucléique ; et
la réalisation d'une opération de correction sur le nombre initial de séquences pour obtenir le nombre de séquences d'acide nucléique dans la donnée d'alignement de chacun de l'au moins un compartiment d'acide nucléique.

6. Procédé selon la revendication 5, dans lequel l'opération de correction comprend au moins l'une parmi une correction de longueur de base efficace, une correction de valeurs aberrantes, une correction de mappabilité ou une correction de teneur en guanine-cytosine (GC).

7. Procédé selon la revendication 1, dans lequel la détermination de la séquence de profondeur de séquençage du chromosome en cours de test selon l'au moins un rapport de profondeur de séquençage de référence, comprend :
en réponse au fait que l'au moins un paramètre de profondeur de séquençage est l'au moins un rapport de profondeur de séquençage linéaire, l'acquisition d'au moins une séquence de rapports de profondeur de séquençage correspondant à au moins un échantillon d'euploïdie, dans lequel chacune de l'au moins une séquence de rapports de profondeur de séquençage comprend au moins un rapport de profondeur de séquençage standard, et pour chaque séquence de rapports de profondeur de séquençage, la séquence de rapports de profondeur de séquençage correspond à un échantillon respectif de l'au moins un échantillon d'euploïdie, chacun de l'au moins un rapport de profondeur de séquençage standard dans la séquence de rapports de profondeur de séquençage correspond à un chromosome respectif de l'au moins un chromosome prédéfini, et le rapport de profondeur de séquençage standard est un rapport entre une profondeur de séquençage du chromosome en cours de test et une profondeur de séquençage du chromosome prédéfini respectif dans l'échantillon d'euploïdie respectif ;
la construction d'une matrice de rapports de profondeur de séquençage selon l'au moins une séquence de rapports de profondeur de séquençage ;
la réalisation d'une optimisation selon la matrice de rapports de profondeur de séquençage et la séquence de compartiments de chromosome pour obtenir au moins un paramètre d'ajustement linéaire correspondant au chromosome en cours de test ; et
la réalisation d'une opération de correction linéaire sur l'au moins un rapport de profondeur de séquençage de référence séparément selon l'au moins un paramètre d'ajustement linéaire pour obtenir l'au moins un rapport de profondeur de séquençage linéaire.

8. Procédé selon la revendication 7, dans lequel des contraintes pour l'optimisation comprennent qu'une valeur absolue d'une différence entre la séquence de profondeur de séquençage et la séquence de compartiments de chromosome est minimale et que chacun de l'au moins un paramètre d'ajustement linéaire comprend une pente qui est supérieure à un seuil positif prédéfini.

9. Procédé selon la revendication 8, dans lequel le rapport de profondeur de séquençage linéaire du chromosome *i* en cours de test et du chromosome *j* prédéfini est représenté par tactᵢⱼ = wᵢⱼ × tᵢⱼ + bᵢⱼ, où wᵢⱼ désigne une pente correspondant au chromosome *i* en cours de test et au chromosome *j* prédéfini*,* et bᵢⱼ désigne un paramètre constant correspondant au chromosome *i* en cours de test et au chromosome *j* prédéfini ;
une somme de |tactᵢⱼ-rᵢⱼ| est minimale et wᵢⱼ est supérieur au seuil positif prédéfini.

10. Procédé selon l'une quelconque des revendications 1 à 9, dans lequel, selon la séquence de compartiments de chromosome et la séquence de profondeur de séquençage, la réalisation du test non paramétrique pour déterminer le résultat de détection d'aneuploïdie du chromosome en cours de test dans l'échantillon d'acide nucléique en cours de test, comprend :
en réponse au fait que le test non paramétrique est un test de permutation, la détermination d'une statistique de test standard selon la séquence de compartiments de chromosome et la séquence de profondeur de séquençage, dans lequel la statistique de test standard est une différence entre une moyenne de séquence de la séquence de compartiments de chromosome et une moyenne de séquence de la séquence de profondeur de séquençage ;
selon un nombre prédéfini de permutations, la réalisation d'une opération d'échange de données sur la séquence de compartiments de chromosome et la séquence de profondeur de séquençage pour obtenir au moins un groupe de séquences de permutation, dans lequel chacun de l'au moins un groupe de séquences de permutation comprend une séquence de compartiments de chromosome permutée respective et une séquence de profondeur de séquençage permutée respective ;
pour chaque groupe de séquences de permutation, la détermination d'une statistique de test de permutation correspondant au groupe de séquences de permutation, dans lequel la statistique de test de permutation est une différence entre une moyenne de séquence de la séquence de compartiments de chromosome permutée dans le groupe de séquences de permutation et une moyenne de séquence de la séquence de profondeur de séquençage permutée dans le groupe de séquences de permutation ; et
la détermination du résultat de détection d'aneuploïdie du chromosome en cours de test dans l'échantillon d'acide nucléique en cours de test selon la statistique de test standard et la statistique de test de permutation.

11. Procédé selon la revendication 10, dans lequel la détermination du résultat de détection d'aneuploïdie du chromosome en cours de test dans l'échantillon d'acide nucléique en cours de test selon la statistique de test standard et au moins une statistique de test de permutation, comprend :
l'utilisation d'une statistique de test de permutation supérieure à la statistique de test standard parmi l'au moins une statistique de test de permutation en tant que statistique de test cible ;
l'utilisation d'un rapport entre un volume de données de la statistique de test cible et le nombre prédéfini de permutations en tant que valeur de probabilité de test ;
en réponse au fait que la valeur de probabilité de test est inférieure à un niveau de signification, le fait de déterminer que le résultat de détection d'aneuploïdie du chromosome en cours de test dans l'échantillon d'acide nucléique en cours de test est une aneuploïdie ; et
en réponse au fait que la valeur de probabilité de test est supérieure ou égale au niveau de signification, le fait de déterminer que le résultat de détection d'aneuploïdie du chromosome en cours de test dans l'échantillon d'acide nucléique en cours de test est une euploïdie.

12. Procédé selon l'une quelconque des revendications 1 à 9, comprenant en outre :
l'extraction d'un acide nucléique libre de l'échantillon d'acide nucléique en cours de test ;
la réalisation d'une amplification par réaction en chaîne par polymérase (PCR) sur l'acide nucléique libre et la réalisation d'un prétraitement d'échantillon pour obtenir une banque d'acides nucléiques ; et
la réalisation d'un séquençage de génome entier sur la banque d'acides nucléiques pour obtenir les données de séquençage de génome entier de l'échantillon d'acide nucléique en cours de test.

13. Appareil permettant de détecter une aneuploïdie chromosomique, comprenant :
un module de détermination de séquence de compartiments de chromosome, qui est configuré pour déterminer une séquence de compartiments de chromosome d'un chromosome en cours de test selon des données d'acide nucléique de génome de référence d'un génome de référence humain, dans lequel la séquence de compartiments de chromosome comprend au moins un rapport de nombre de compartiments rᵢⱼ, et chacun de l'au moins un rapport de nombre de compartiments est un rapport rᵢⱼ = Lᵢ/Lⱼ, où *i ≠ j,* entre un nombre Lᵢ de compartiments d'acide nucléique du chromosome *i* en cours de test pour une aneuploïdie dans le génome de référence humain et un nombre Lⱼ de compartiments d'acide nucléique d'un chromosome respectif d'au moins un chromosome *j* prédéfini qui n'est pas en cours de test pour une aneuploïdie dans le génome de référence humain ;
un module de détermination de séquence de profondeur de séquençage, qui est configuré pour déterminer une séquence de profondeur de séquençage du chromosome *i* en cours de test selon des données de séquençage de génome entier d'un échantillon d'acide nucléique en cours de test, dans lequel la séquence de profondeur de séquençage comprend au moins un paramètre de profondeur de séquençage, et chacun de l'au moins un paramètre de profondeur de séquençage est soit un rapport de profondeur de séquençage de référence tᵢⱼ = Hᵢ/Hⱼ, où i ≠ j, entre une profondeur de séquençage Hᵢ du chromosome i en cours de test dans l'échantillon d'acide nucléique en cours de test et une profondeur de séquençage Hⱼ d'un chromosome respectif de l'au moins un chromosome *j* prédéfini non en cours de test dans l'échantillon d'acide nucléique en cours de test, soit un rapport de profondeur de séquençage linéaire qui est basé sur le rapport de profondeur de séquençage de référence ; et
un module de détermination de résultat de détection d'aneuploïdie, qui est configuré pour, selon la séquence de compartiments de chromosome et la séquence de profondeur de séquençage, réaliser un test non paramétrique pour obtenir un résultat de détection d'aneuploïdie du chromosome en cours de test dans l'échantillon d'acide nucléique en cours de test.

14. Support de stockage lisible par un ordinateur permettant de stocker une instruction informatique, dans lequel l'instruction informatique, lorsqu'elle est exécutée par un processeur, amène le processeur à réaliser le procédé permettant de détecter une aneuploïdie chromosomique selon l'une quelconque des revendications 1 à 12.
